# EUROPEAN PATENT APPLICATION

(11) **EP 3 795 172 A1**
(43) Date of publication of application: **24.03.2021**
(21) Application number: 19198817.9
(22) Date of filing: 20.09.2019
(51) Int. Cl.: A61K 39/00, A61K 39/39

(54) **POLYMER OR POLYCONDENSATE BASED ON PEPTIDE, LINKER AND OPTIONALLY OTHER MONOMERS AND METHOD FOR PREPARING THE SAME**

(71) Applicant: Ecole Polytechnique Federale De Lausanne (EPFL) EPFL-TTO, 1015 Lausanne (CH)
(72) Inventor: TANG, Li, 1112 Echichens VD (CH); WEI, Lixia, 1025 St-Sulpice (CH); ZHAO, Yu, 1007 Lausanne (CH)
(74) Representative: Omnis-IP

(57) **Abstract**

The present invention concerns a method for producing polymers suitable as carrier-free delivery system of bioactive molecules, in particular bioactive peptides. The method comprises the polycondensation of one or more different monomers, including monomers comprising the peptides, monomers comprising a cross-linker and, optionally, monomers comprising an adjuvant. The invention also relates to the polymers obtained by the method, which are suitable as medicaments, such as vaccines. In a particular embodiment, the bioactive peptide is a neoantigen and the polymer is used as a cancer vaccine.

SEQUENCE LISTING

<110> EPFL

<120> Polymer or Polycondensate Based on Peptide, Linker and Optionally
Other Monomers

<130> P-1000-002-EP

<160> 15

<170> BiSSAP 1.3.6

<210> 1
<211> 18
<212> PRT
<213> Artificial Sequence

<220>
<223> Synthetic construct

<400> 1
Leu Glu Gln Leu Glu Ser Ile Ile Asn Phe Glu Lys Leu Lys Lys Lys
1               5                   10                  15
Lys Lys

<210> 2
<211> 21
<212> PRT
<213> Artificial Sequence

<220>
<223> Synthetic peptide

<400> 2
Lys Lys Lys Lys Lys Leu Glu Gln Leu Glu Ala Ala Tyr Ser Ile Ile
1               5                   10                  15
Asn Phe Glu Lys Leu
            20

<210> 3
<211> 8
<212> PRT
<213> Gallus gallus

<220>
<223> CD8 epitope of ovalbumin

<400> 3
Ser Ile Ile Asn Phe Glu Lys Leu
1               5

<210> 4
<211> 5
<212> PRT
<213> Artificial Sequence

<220>

<223> Polylysine tag

<400> 4
Lys Lys Lys Lys Lys
1               5

<210> 5
<211> 5
<212> PRT
<213> Artificial Sequence


<220>
<223> Sequence flanking neoepitope 1

<400> 5
Leu Glu Gln Leu Glu
1               5

<210> 6
<211> 8
<212> PRT
<213> Artificial Sequence


<220>
<223> Sequence flanking neoepitope 2

<400> 6
Leu Glu Gln Leu Glu Ala Ala Tyr
1               5

<210> 7
<211> 4
<212> PRT
<213> Artificial Sequence


<220>
<223> Polylysine tag of PAM3CSK4

<400> 7
Lys Lys Lys Lys
1

<210> 8
<211> 9
<212> PRT
<213> Mus musculus


<220>
<223> MC38 murine colorectal cancer epitope, also known as adpgk

<400> 8
Ala Ser Met Thr Asn Met Glu Leu Met
1               5

<210> 9
<211> 9
<212> PRT
<213> Mus musculus

<220>
<223> B16F10 murine menaloma epitope, also known as TRP-2 (180 - 188)

<400> 9
Ser Val Tyr Asp Phe Phe Val Trp Leu
1               5

<210> 10
<211> 9
<212> PRT
<213> Mus musculus


<220>
<223> M27 neoantigen from B16F10 melanoma

<400> 10
Leu Cys Pro Gly Asn Lys Tyr Glu Met
1               5

<210> 11
<211> 15
<212> PRT
<213> Artificial Sequence


<220>
<223> Peptide comprising adpgk neoepitope

<400> 11
Cys Ala Ser Met Thr Asn Met Glu Leu Met Lys Lys Lys Lys Lys
1               5                   10                  15

<210> 12
<211> 20
<212> PRT
<213> Artificial Sequence


<220>
<223> Peptide comprising adpgk neoepitope

<400> 12
Glu Leu Glu Lys Lys Lys Lys Lys Ala Ala Tyr Ala Ser Met Thr Asn
1               5                   10                  15
Met Glu Leu Met
            20

<210> 13
<211> 15
<212> PRT
<213> Artificial Sequence


<220>
<223> Peptide containing TRP-2 (180 - 188) neoepitope

<400> 13
Cys Ser Val Tyr Asp Phe Phe Val Trp Leu Lys Lys Lys Lys Lys
1               5                   10                  15

<210> 14

<211> 15
<212> PRT
<213> Artificial Sequence

<220>
<223> Peptide M27 neoantigen from B16F10 melanoma

<400> 14
Cys Leu Cys Pro Gly Asn Lys Tyr Glu Met Lys Lys Lys Lys Lys
1               5                   10                  15

<210> 15
<211> 20
<212> DNA
<213> Artificial Sequence

<220>
<223> CpG polydesoxynucleotide

<400> 15
tccatgacgt tcctgacgtt                                                   20

## Description

### Technical Field

The present invention relates to polymers and/or polycondensates based on peptide, linker and other types of monomers. The polymer molecules of the invention may be used for therapeutic and/or prophylactic medical purposes, for example for vaccination.

### Background Art and Problems Solved by the Invention

Therapeutic cancer vaccines designed to induce or augment anticancer T cell response have shown clinical benefit in many phase I/II studies. However, the clinical efficacy of cancer vaccines remain modest in comparison with other immunotherapies such as checkpoint blockade and adoptive T cell therapy. Cancer vaccines targeting antigens derived from random somatic mutations in tumor cells but not present in normal cells, termed neoantigens, have recently been developed as a personalized immunotherapy and shown great promise in the treatment of late stage melanoma. Compared to self-antigens, neoantigens could be recognized as non-self by the host immune system and are thus attractive targets for immunotherapies with potentially increased specificity, efficacy, and safety. However, the elicited anticancer T cell response by neoantigen-based vaccines is in general weak and short-lived partially due to the lack of a versatile and highly effective delivery platform that can be facilely adapted for vastly diverse neoepitopes identified from individual patients.

There are two major challenges in the delivery of peptide-based cancer vaccines. One is targeting antigens in tandem with suitable adjuvants to secondary lymphoid organs, such as lymph nodes (LNs), to facilitate robust antigen capture and processing by professional antigen presenting cells (APCs), such as dendritic cells (DCs). Parenterally injected soluble peptide antigens or adjuvant molecules typically disseminate into systemic circulation due to the small sizes and show very poor uptake in LNs resulting in limited immune responses. Moreover, soluble molecular adjuvants administered subcutaneously often result in significant systemic inflammatory toxicities. The other major challenge is to elicit robust cytotoxic CD8⁺ T cell responses, which is essential for eradicating tumor cells through orchestrating with CD4⁺ T cell responses. Soluble subunit antigens acquired by DCs from the extracellular environment are internalized into endolysosomal compartments and loaded almost exclusively onto major histocompatibility complex (MHC) class II molecules for the presentation to CD4⁺ helper T cells. Typically, only antigens located in the cytosol can be loaded onto MHC class I molecules for the presentation to CD8⁺ killer T cells, a process termed cross-presentation. Thus, it is critical to control the intracellular pathways of captured antigens for enhanced cross-presentation.

It is a general objective of the invention to provide a novel platform for the delivery of bioactive molecules. It is in particular an objective to provide a platform that is designed to release bioactive compounds under particular conditions only and/or in a particular chemical environment, for example under reducing conditions or under acidic conditions, or both, for example. By allowing to define the conditions of release, the release at a particular site of interest can be improved.

It is an objective of the invention to provide a delivery system or platform for delivering bioactive molecules, such as antigens and/or immunogens, for example in vaccination therapies, including but not limited to cancer vaccines.

It is an objective of the invention to provide a delivery system or platform for delivering peptide-based molecules in the human or animal body, preferably to a particular target within the body, such as a particular organ or particular cells.

It is an objective of the invention to provide a carrier-free platform for delivering bioactive molecules to the human or animal body, preferably in a targeted manner.

It is an objective of the invention to provide a delivery system or platform for delivering a combination of two or more different, preferably synergistically acting, bioactive molecules, to the human or animal body, and achieving simultaneous release of the bioactive molecules. For example, it is an objective of the invention to provide a delivery system that allows targeting to particular cells or organs.

It is a further objective of the invention to provide a delivery system or platform that exhibits high loading capacity and high incorporation efficiency of the bioactive molecules.

It is an objective of the invention to provide a delivery system or platform having properties that result in accumulation in the lymph nodes through the EPR effect (Enhanced Permeability and Retention). It is an objective to provide a vaccine having the appropriate particle size, such as preferably in the range of 10-100 nm.

It is an objective of the invention to provide a delivery system or platform that releases the bioactive molecule without leaving any trace on the bioactive molecule "traceless release". It is an objective to deliver the bioactive molecule in its original form. In particular, it is an objective to provide a bioactive molecule, such as a bioactive peptide, without any substituents or residues related to the delivery system used. The traceless delivery of a bioactive molecule, such as an antigen in the case of a vaccine, is an objective of the invention.

It is a further objective of the invention to provide a delivery system or platform that does not involve a matrix or a core-shell particle that is left over after delivery. For example, is an objective to provide a delivery vehicle of which all components will be metabolized and/or excreted by the organism.

US 2015/0110740 discloses protein-polymer nanogels comprising degradable, redox-responsive linkers. Under reducing conditions, the linkers of the nanogel release the proteins, such as cytokines, growth factors, antibodies or antigens, of the protein polymer. In view of this reference, it is an objective to provide different macromolecules, such as polymers comprising different monomers and polymers that enable release under other conditions, for example. It is also an objective of the invention to provide polymers and/or polycondensates of smaller monomeric units, such as oligo- or small polypeptides instead of only large proteins.

US 2019/0060463 discloses bridged polyethylene glycol-aliphatic polyester block copolymer, which are conceived for the delivery of chemotherapeutic drugs and nucleic acid drugs. The copolymers comprise an amide bond that responsive to pH in tumor cells. Active principles such as hydrophobic drugs or siRNA is entrapped within a hydrophobic core of the nanoparticles formed by the copolymer. In view of this reference, it is an objective to provide a delivery system for peptides and/or water-soluble molecules. It is also an objective of the invention to provide a platform that allows the release of the active principles under other conditions, such as under reducing conditions and/or under acidic conditions.

The present invention addresses the problems depicted above.

### Summary of the Invention

Remarkably, the present inventors provide polymers, in which bioactive molecules are integrated in the polymer. The polymers provide a delivery vehicle obtained by reacting optionally derivatized bioactive molecules with linker molecules imparting particular release characteristics to the delivery vehicle. The obtained delivery vehicle is preferably a carrier-free and shell-free delivery vehicle, as the bioactive molecules are preferably integrative part of the delivery vehicle, which is designed to decompose and release constituents under specific, predetermined conditions.

In an aspect, the invention provides a method of obtaining a polymer, the method comprising reacting at least one of first monomers (type A monomers) selected from monomers of formula (1):

A₁⁅G]*_{q}* (1)

and optionally from y different further monomers of formula (1'),

A_{X}⁅G]ᵣ₍ₓ₎ (1'),

with second monomers of formula (2) (type B or linker monomer)

D-L-D (2),

wherein
A₁ comprises a peptide,
y is 0 (if there is no monomer (1'), 1 or an integer larger than 1;
X is an integer of 2 and up to y+1, wherein there are y monomers of formula (1'), such that X is a successive integer from 2 and up to y+1 for each additional monomer (A₂, A₃, A₄, ... A_{y+1});
Ax is selected from molecules comprising at least one functional group G, including from molecules comprising peptides, wherein A₁ and said y A_{X} are different monomers;
G are free functional groups of peptides A₁ and of A_{X};
q and r(x) represent the total numbers of free functional groups G of A₁ and of said one or more A_{X}, respectively, wherein q and r(x) are independently 1 or more than 1;
L is an organic linker moiety comprising from 1 to 1000 carbons and from 0 to 300 heteroatoms,
D is a free functional group of said type B monomer,
wherein during said step of reacting, said groups D reacts with at least some of said groups G so as to connect said first and second monomers.

In an aspect, the invention provides a method for producing polymers that decompose in response to a particular pH change or value, preferably under acidic pH, the method comprising reacting molecules, preferably peptides, comprising one or more free amino groups (such as said type A monomers), with linker monomers of formula (2) comprising functional groups of formula (5) and preferably linker moieties L of formula (10).

In an aspect, the invention provides a method for cross-linking bioactive compounds comprising free amino groups, the method comprising reacting said bioactive compounds, with linker monomers of formula (2) comprising functional groups of formula (5) and preferably linker moieties L of formula (10).

In some aspects, the invention provides novel compounds, such as compounds of formula (2)

D-L-D (2)

wherein D is a group of formula (5) and L is a moiety of formula (10) as defined in this specification.

In some aspects, the invention provides novel compounds, such as compounds of formulae (28), (29), (31) and (32).

In an aspect, the invention provides the use of monomers of formula (2):

D-L-D (2)

and molecules comprising one or more free amino groups for producing polymers that decompose in response to pH, preferably under acidic pH, wherein D is a group of formula (5) and L is a moiety of formula (10) as defined in this specification.

In an aspect, the invention provides the use of monomers of formula (2):

D-L-D (2)

wherein D is a group of formula (5) and L is a moiety of formula (10) as defined in this specification, for cross-linking bioactive compounds comprising free amino groups.

In an aspect, the present invention provides the use of compounds of formula (2) comprising functional groups of formula (5) and preferably linker moieties L of formula (10) for producing a pH responsive delivery system.

In an aspect, the invention provides a polymer comprising one or several moieties of formula (40) and or (40a): 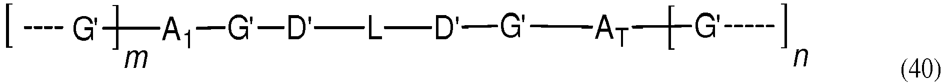 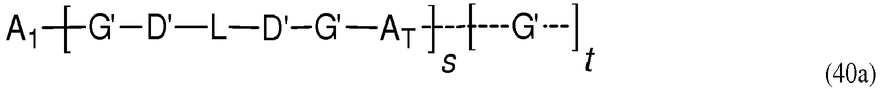 wherein:
A₁ comprises a peptide;
L is an organic linker moiety comprising from 1 to 1000 carbons and from 0 to 300 heteroatoms;
T is 1 or 2, wherein if T is 2, A_{T} is A₂ which is different from A₁;
if T is 2, A₂ is a molecule comprising one or more (n+1) functional groups G;
the moieties -G'-D'- and -D'-G'- are moieties obtained by the reaction of groups D with groups G as defined in with respect to formula (1) and (1') above;
wherein m and n independently represent the numbers of groups G of A₁ and A_{T} that have reacted with groups D of the second monomer of formula (2), wherein m and n are independently equal or smaller than p and n as defined with respect to formulae (1) and (1'), preferably 100 or less. s = m+1 and t = n.

Further aspects and preferred embodiments of the invention are defined herein below and in the appended claims.

Further features and advantages of the invention will become apparent to the skilled person from the description of the preferred embodiments given below. These embodiments are provided by way of examples and the present invention is not intended to be limited to the particular embodiments as illustrated in the figures.

### Brief Description of the Drawings

**Figures 1A** and **1B** are schematic illustrations of the synthesis of polymers according to different embodiments of the invention, respectively.
**Figure 1C** is a schematic illustration of the pH responsive release of polymers shown in Fig. 1B
**Figure 2A** shows retention time in gel permeation chromatography (GPC) of PNE(LEQ-Pam) and of LEQ, which are polymers according two different embodiments of the invention (see Examples 1 and 2 in Fig. 10). LEQ refers to the monomer LEQLESIINFEKLKs (SEQ ID NO: 1), and Pam is Pam₃CSK₄. These polymers were prepared using a redox-sensitive linker as described in the examples.
**Figure 2B** and **2C** show size distribution measurement by dynamic light scattering (DLS) and atomic force microscopy (AFM) imaging a, respectively, of PNE(LEQ-Pam) (Fig. 2A).
**Figure 2D** illustrates the scheme of the redox-responsive release of antigens and adjuvants from PNE(LEQ-Pam).
**Figure 2E** shows the MALDI-TOF mass spectrometric analysis of native and released LEQ from PNE(LEQ-Pam).
**Figure 2F** shows the release kinetics of LEQ (Fig. 2A) from PNEs in the presence or absence of a reducing agent DTT (2 mM).
**Figure 3A** and **3B** show IVIS fluorescent imaging and quantification, respectively, of inguinal draining lymph nodes (LNs) excised from mice injected with different fluorescently labeled vaccines in accordance with embodiments of the invention. PNE(LEQ-Pam) in the right lane shows strongest fluorescence and thus highest accumulation in LNs following subcutaneous injection into C57BL/6 mice.
**Figure 3C** shows antigen capture by dendritic cells DCs in the inguinal LNs, quantified with flow cytometry analysis. DCs of mice vaccinated with PNE(LEQ-Pam), according to a preferred embodiment, showed highest Median Fluorescence Intensity (MFI).
**Figure 4** shows counts of proliferated OT-1 CD8+ T cells co-cultured with BMDCs pulsed with PNE- or other indicated formulations. BMDCs pulsed with PNE(LEQ-Pam) cross-primed the SII-specific naive OT-1 CD8⁺ T cells with a greatly enhanced efficiency compared to monomeric SII or LEQ, or the simple mixture of short or long peptides with Pam, assessed by a 5(6)-carboxyfluorescein diacetate *N*-succinimidyl ester (CFSE) dilution assay. SII is the peptide SIINFEKL (SEQ ID NO: 3) **P* < 0.05; ***P* < 0.01; ****P* < 0.001; *****P* < 0.0001.
**Figure 5A** illustrates the experimental time line of the immunization of mice with PBS, LEQ+Pam, (LEQ+Pam)-Montanide and PNE(LEQ-Pam) vaccine.
**Figure 5B** **and** **5C** show representative flow cytometry plots and the frequencies, respectively, of SIINFEKL (SEQ ID NO: 3)-specific CD8⁺ T cells among all the CD8⁺ T cells in peripheral blood sampled on Day 21, in accordance with the experimental setting shown in Fig. 5A.
**Figure 6A** shows the release kinetics of PNE_{pH}(K₅LEQ) under various pH values.
**Figure 6B** illustrates the scheme of the breakage of the linker ("monomer B") from the first monomer ("monomer A") in dependence of pH in polymers (on the left side) designed for the pH-responsive release of peptides, resulting in the release in the peptide monomers (on the right side).
**Figures 7A** and **7B** show IVIS fluorescent imaging and quantification, respectively, of inguinal draining lymph nodes (LNs) excised from mice injected with different fluorescently labeled vaccines in accordance with embodiments of the invention, including the pH-sensitive PNE_{pH}(K₅LEQ-Pam)-1.
**Figure 7C** shows antigen capture by dendritic cells DCs in the inguinal LNs, quantified with flow cytometry analysis. The vaccines that were tested are as shown in the figure and include a pH-sensitive PNE_{pH}(K₅LEQ-Pam)-1.
**Figure 8A** illustrates the experimental time line of the immunization of mice with PBS, KsLEQ+Pam, and several different pH-sensitive vaccine polymers according to embodiments of the invention PNE_{pH}(K₅LEQ-Pam)-1, PNE_{pH}(K₅LEQ-Pam)-2, and PNE_{pH}(K₅LEQ-CpG)-1.
**Figure 8B** shows representative flow cytometry plots of SIINFEKL (SEQ ID NO: 3)-specific CD8⁺ T cells among all the CD8⁺ T cells in peripheral blood sampled on Day 28.
**Figure 8C** shows representative flow cytometry plots of re-stimulated splenocytes on D40, following the experimental outlined in Fig. 8A.
**Figure 8D** shows frequency (left graph) and cell counts (right graph) of SIINFEKL (SEQ ID NO: 3)-specific CD8⁺ T cells among all the CD8⁺ T cells in re-stimulated splenocytes of LN on Day 40, following the experiment set out in Fig. 8A.
**Figures 9** and **10** are tables showing various different polymers produced in accordance with the invention.
**Figure 11** shows the sequence ID numbers (SEQ ID NO) in the sequence listing of amino acid sequences disclosed in the present specification.

### Detailed Description of the Preferred Embodiments

The present invention relates to polymers, wherein molecules to be delivered are preferably integrated in the polymer, and to methods for obtaining the polymers. Said polymers are preferably branched polymers, forming preferably an overall network structure and/or a macromolecule obtained by cross-linking molecules to be delivered. In this specification, the term "polycondensate" may be used to refer to the molecules of the invention. In the particular example where the molecule to be delivered comprises a neoepitope, the term "polycondensates neoepitope" (PNE) may be used, for example in the examples-section, to refer to the compounds encompassed by the present invention.

The molecules to be delivered are preferably bioactive molecules, for examples moieties A₁ and A_{X} in formulae (1) and (1'). These molecules preferably comprise, or were derivatized to comprise, one or more functional groups G that allow the condensation of the molecules with preferably bifunctional linker monomers. Each linker monomer preferably comprises two groups D, which can react with said groups G, so as to form the polymer.

The method of the invention is preferably a condensation reaction, where reactive groups D of said linker monomer reacts with the functional group G, thereby providing a two- or preferably three-dimensional network composed of bioactive monomers A₁ and optionally one or more A_{X}, and said linker monomers. Preferably, the polymers form separate particles. Preferably, said particles are nanoparticles. The number of functional groups G and the number of linker monomers will determine the extent of cross-linking.

During the polycondensation reaction, linker monomers (monomers B) of formula (2) can react with at most two different monomers comprising moiety A₁ and optionally one or more A_{X} (monomers A) of formula (1) and optionally (1'), but each monomer A₁, A_{X}, etc, can react with one, two, three four or potentially more linker molecules, resulting in an overall complex polymeric or polycondensate structure.

Preferably, said polymer, and in particular said linker monomer, is designed so as to break apart or disintegrate under predefined conditions, thereby releasing the monomer moieties A₁ and optionally one or more A_{X}, and more precisely the monomers of formula (1) and/or (1'), in their original forms. Preferably, said disintegration may take place in dependence of pH and/or in a reducing environment. As discussed elsewhere in this specification, the release of A₁ and optionally one or more A_{X} is preferably traceless.

In an embodiment, A₁ comprises a peptide. Said peptide preferably provides said bioactive molecule and is thus a bioactive peptide. Said peptide may be an oligopeptide and/or a polypeptide. In an embodiment, said bioactive peptide comprises an oligo- or polypeptide consisting of 2 to 2000, preferably 5 to 1000, more preferably 5 to 500 amino acids. In a preferred embodiment, said bioactive peptide comprises an oligo- or polypeptide consisting of 2 to 150, preferably 5 to 100, most preferably 5 to 50 amino acids.

For the purpose of the present specification, an oligopeptide has from 2 to 10 amino acids and a polypeptide more than 10 amino acids.

In an embodiment, said peptide comprised in A₁ comprises an antigen and/or an epitope (an antigenic determinant).

An exemplary epitope is the SIINFEKL neoepitope (SEQ ID NO: 3), the CD8 epitope of ovalbumin.

Preferably, said antigen and/or epitope is suitable to elicit an immune response in a human or animal subject, to which the antigen/epitope was administered, possibly if co-administered with a suitable adjuvant. If A₁ comprises an immunogenic determinant, the polymer of the invention is preferably a vaccine and/or may preferably be used as a vaccine.

In an embodiment, said bioactive peptide comprises an oligo- or polypeptide comprising an immunogenic determinant, preferably a neoantigen.

In a particular embodiment, said antigen is a neoantigen. A neoantigen is preferably an antigen derived from a random somatic mutation in a tumor cell. In this case, the polymer is preferably a cancer vaccine.

Exemplary neoantigens are: ASMTNMELM (SEQ ID NO: 8), also known as "adpgk" is a neoeptitope from MC38 murine colon carcinoma cells. It can also be found in B16F10 cells. SVYDFFVWL (SEQ ID NO: 9), also known as "TRP-2 (180-188)", is a neoepitope from B16F10 melanoma. LCPGNKYEM (SEQ ID NO: 10) is the M27 neoantigen from B16F10 melanoma (Adv. Therap. 2018, 1, 1800060).

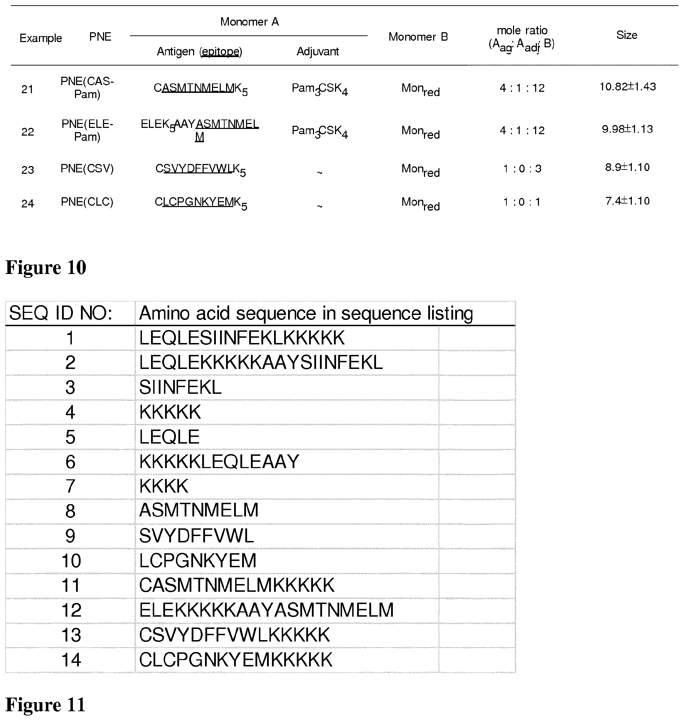

Further exemplary type A monomers, from which A₁ may be selected, are the peptides with sequence numbers SEQ ID NO: 1-3, 8-10 and 11-14. Sequences with SEQ ID NO: 4-7 concern sequences that may be comprised in A₁, such as oligo- or polylysine lysine tags (SEQ ID NO: 4 and 7) and further tags, flanking amino acids or peptides and the like (SEQ ID NO: 5, 6). Amino acid sequences SEQ ID NOs: 11-14 (see the table in Fig. 10) are synthetic peptides comprising the neoepitopes of SEQ ID NO: 8-10 and one or two flanking sequences, such as in particular oligolysine tags.

In accordance with the invention, the polymer of the invention may be used as a vaccine, for example a cancer vaccine, even if a neoantigen is absent. Instead, another antigen known to induce a protective immune response against cancer cells may be used, for example.

A₁ is not limited to bioactive molecules comprising an antigenic determinant. A₁ may comprise another bioactive molecule, for example a cytokine, and antibody, and so forth. The invention is not limited to moieties A₁ having a particular bioactive function, but may be worked using any suitable peptide having a desired bioactive activity.

Said moieties A₁ and, if present, A₂, A₃, A₄, ... , A_{y} comprise a functional group G. In an embodiment, the functional groups G of the moiety A₁ and/or A_{X} comprise and/or consist essentially of a free amino group (-NH₂).

If A₁ and/or A_{X} comprise a peptide, G may be an amino acid side chain functional group. In an embodiment, the functional group is side chain amino group (-NH₂). In a preferred embodiment, the functional group is a side chain amino group of the amino acid lysine. If the moieties A₁ and/or A_{X} comprise a peptide, the amino group may also be the amine group connected to the α-carbon of the amino acid, in particular the amino group of the N-terminal amino acid of the peptide.

In an embodiment, A₁ and/or A_{X} further comprise a terminal lysine or a peptide tag comprising one or several lysines. Preferably, at least one of said groups G, respectively, is present on said peptide tag and is an amino side chain group of said lysine or lysines.

The tag may be a peptide tag comprising several consecutive lysines or lysines separated by other amino acids. For example, the tag may comprise a polylysine and/or an oligolysine. Preferably, one or more of said groups G, respectively, is present on said peptide tag and is an amino side chain groups of said lysine or lysines.

Altogether, including one or more tags and a bioactive peptide, A₁ preferably comprises 2 to 2000, preferably 5 to 1000, more preferably 5 to 500 amino acids, thus including the bioactive peptide and the tag. In a preferred embodiment, A₁ comprises 5 to 120, preferably 10 to 100, most preferably 12 to 60 amino acids.

In an embodiment, A₁ and/or A_{X}, independently, may comprise an oligolysine, a polylysine, or a tag comprising one or more lysine moieties, preferably in addition to said bioactive peptide.

One or more A_{X}, for example A₂, may be selected, independently, from the moieties A₁ as defined herein above. Preferably, A_{X} are different from A₁. Monomers of formula (I') may be absent. Any A_{X} is independently more generally selected from molecules comprising at least one functional group G, preferably one or several free amine groups, when A_{X} is present as a free monomer.

In a preferred embodiment, one or more A_{X} are selected from moieties comprising an adjuvant, preferably a molecular adjuvant.

In an embodiment, said adjuvant is an organic molecule comprising from 1 to 200 carbons and 1-100 heteroatoms. More preferably, said adjuvant is a molecular adjuvant comprising from 5 to 150 carbons and from 2 to 50 heteroatoms. Heteroatoms are preferably selected from O, N, S, and halogen, preferably from O, N, and S.

In a preferred embodiment, y is 1, larger than one and up to 500, preferably up to 200, most preferably up to 100. Preferably, y is an integer of 0 to 50, more preferably 1 to 20. In a preferred embodiment, y is 1, 2, 3, 4, 5, 6, 7, 8, 9, or 10.

In an embodiment, said y A_{X} are independently selected from the moieties comprising peptides and further from moieties comprising an adjuvant.

In an embodiment, said adjuvant is selected from the group consisting of: Pam₃CSK₄, cGAMP, LPS, flagellin, MPLA, cytosine-phosphate-guanosine (CpG), (for example CpG oligo-and/or polydeoxynucleotide), Poly(I:C), Poly ICLC, Imidazoquinoline, R848 and all kinds of molecular adjuvants. In a preferred embodiment, A_{X} (e.g. A₂) is selected from Pam₃CSK₄, CpG oligo-and/or polydeoxynucleotide, such as CpG-bisamine and CpG-monoamine.

Preferably, said adjuvant is amino-functionalized, that is, synthetically modified to comprise one or more additional amine groups.

As can be seen from this list, Pam₃CSK₄ comprises a tetralysine tag, which is why it can be used as a monomer A_{X} (e.g. A₂) by way of the four primary amine groups available in this molecule. Amine-functionalized CpG oligo-or polydeoxynucleotide can also be used as a monomer A_{X}. For example, said amine functionalized CpG polydeoxynucleotide is: 5'-/5AmMC6/TCCATGACGTTCCTGACGTT/3AmMO/-3' (SEQ ID NO: 15).

In case of a vaccine for humans, CpG oligo-or polydeoxynucleotides that are more effective in humans are preferably used.

In an embodiment, there is at least one A_{X} (A₂), which is selected from moieties comprising an adjuvant. A₂, when present as a free monomer, comprises or is derivatized to comprise one or more free amine groups. In this case, A₁ is preferably a bioactive peptide, preferably comprising an antigenic determinant, and A₂ is a molecule comprising an adjuvant.

In a preferred embodiment, two type A monomers are used in the method of the invention, such that y=1 and X=2, A_{X} thus being A₂. Preferably, in this embodiment, A₁ comprises a bioactive peptide and A₂ comprises an adjuvant, for example as disclosed herein above.

In another embodiment, three type A monomers are used in the method of the invention (A₁, A₂, A₃, such that y=2 and X=2, 3. Preferably, in this embodiment, A₁ comprises a bioactive peptide and A₂ comprises an adjuvant, for example as disclosed herein above, and A₃ comprises one selected from a further bioactive peptide and/or a further adjuvant.

In another embodiment, four type A monomers are used in the method of the invention (A₁, A₂, A₃, A₄, such that y=3 and X=2, 3, 4. Preferably, in this embodiment, A₁ comprises a bioactive peptide, A₂ comprises an adjuvant as disclosed herein above, and A₃ and A₄ are independently selected from a further bioactive peptides and/or a further adjuvants, for example as disclosed herein above.

Preferably, if y≥1, and A_{X} is different from A₁ and from any other A_{X}, as present, e.g. A₂ is another molecule than A₁, and A₃ is another molecule than A₁ and A₂.

The second monomers (type B or linker monomer) preferably have the structure of formula (2):

D-L-D (2),

wherein L and D are as defined elsewhere in this specification.

Preferably, D is a free functional group that suitable to react with group G, in particular with a free amine group, so as to create a covalent bond between the type A and type B monomers. Preferably, such a covalent bond (and thus the polymer of the invention) is stable under physiological conditions and RT (25°C). Physiological conditions are preferably defined by a temperature of 25°C (RT), pH of 7.4, in phosphate-buffered saline.

In a preferred embodiment, said phosphate buffered saline is preferably defined by the following constituents: 137 mmol/L NaCl, 2.7 mmol/L KCl, 10 mmol/LNa₂HPO₄, and 1.8 mmol/L KH₂PO₄. 2.84mM NaCl may be added to shift the pH to 7.4.

Preferably, such a covalent bond is readily created under the reaction conditions, as set out elsewhere in this specification.

L is an organic linker moiety comprising from 1 to 1000 carbons and from 0 to 300 heteroatoms, preferably 2 to 500 carbons and 0 to 200 heteroatoms, more preferably 1 to 100 carbons and 1 to 50 heteroatoms, most preferably 2 to 20 carbons and 0-5 heteroatoms.

In a preferred embodiment, L comprises 2 to 10 carbons and 0-5 heteroatoms.

In a particularly embodiment, L comprises 4 carbons and 2 O or S heteroatoms.

Preferably, said heteroatoms, for example as optionally present in L, are selected from O, S, N and halogen, more preferably from O, S and N, most preferably from O and S.

In an embodiment, D is selected from the groups of formula (5), (5') and (6) below: 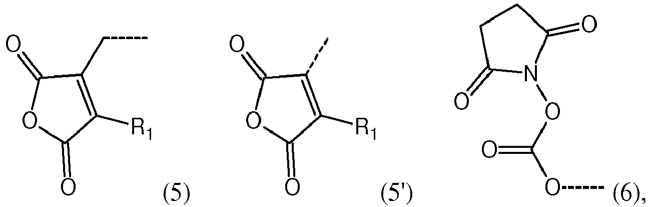
wherein the dotted line is the single bond by which the group is connected to a carbon or heteroatom of L, said heteroatom of L being preferably selected from N, S, and O;
R₁ is selected from H and from aliphatic or aromatic substituents comprising from 1 to 20 carbon atoms and from 0 to 10 heteroatoms. Heteroatoms of R₁ are preferably selected from O, S, N and halogen, preferably from O, N and S, preferably from O and S. In a preferred embodiment, R₁ is selected from alkyl, for example from C1-C20 alkyl, more preferably from C1-C10, most preferably from C1-C5 alkyl. Preferably, said alkyl is linear.

When D has the structure of formula (5) or (5'), the polypeptide obtained by the method of the invention is preferably a pH-responsive linker as disclosed elsewhere in this specification. In this case, L preferably is or comprises a moiety of formula (10).

In an embodiment, L is or comprises a moiety of formula (10): 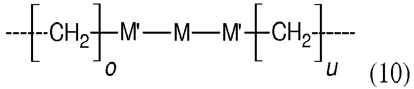 wherein,
o and u are selected, independently, from 0, 1, and 2;
the dotted lines in the structure of formula (10) represent the single bonds by which the carbon of the corresponding CH₂ or M' moiety is connected to said groups D;
o and u are selected, independently, from 0, 1, and 2;
M is an organic moiety comprising from 1 to 1000 carbons and from 0 to 300 heteroatoms;
M' is selected from the moieties of formula (11), (12), (13), (14) and (15) below: 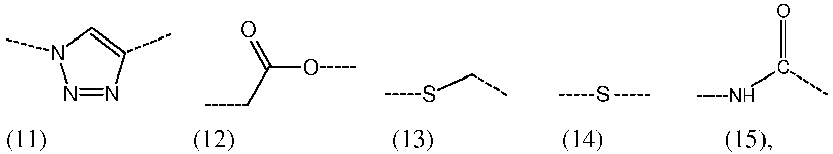 wherein, in formulae (11) to (15), the dotted line on the right represents the bond to M and the dotted line on the left represents the bond to the -CH₂- moiety in formula (10).

Preferably, o and u are 0 or 1, most preferably 0, in particular of D is (5).

The definition of L being a moiety of formula (10) applies preferably where D has the structure of formula (5) or (5').

Preferably, M is a moiety an organic moiety comprising from 1 to 500 carbons and from 0 to 200 heteroatoms, more preferably comprising from 1 to 100 carbons and from 0 to 50 heteroatoms, most preferably from 2 to 8 carbons and 1 to 5 heteroatoms. Said heteroatoms are preferably selected from O, S and N, preferably O and S, most preferably O.

In a preferred embodiment, M is a moiety selected from the moieties of formulae (20)-(23) below: 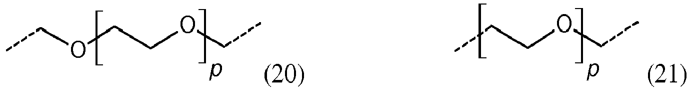 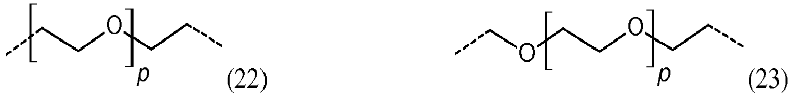 wherein p is 0 or an integer of 1-500, preferably 1-200, more preferably 1-100, even more preferably 1-20. In an embodiment, p is 2-10.

In a preferred embodiment, L is moiety (20), preferably if D is a moiety of formula (5) or (5').

In some embodiments, L comprises a disulphide, preferably provided for allowing disconnection of A₁ and any A_{X} (in as far as present) under reducing conditions, preferably in connection with a self-immolative reaction.

In an embodiment, L is a moiety of formula (25): 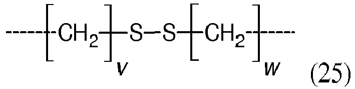 wherein v and w are independently selected from integers of 1-50, preferably 1-10, more preferably 2-5, most preferably v and w are 2.

If L is a moiety of formula (25), the polypeptide obtained by the method of the invention is preferably a redox-responsive linker as disclosed in more detail elsewhere in this specification.

The definition of L being a moiety of formula (25) applies preferably where D has the structure of formula (6).

In an embodiment, said second monomers (type B or linker monomer) are selected from compounds of formula (28) and (29): 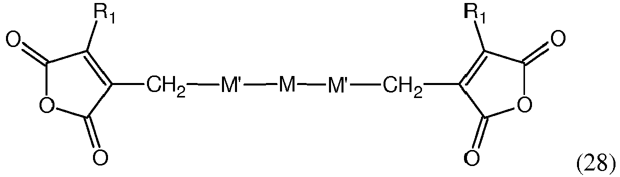 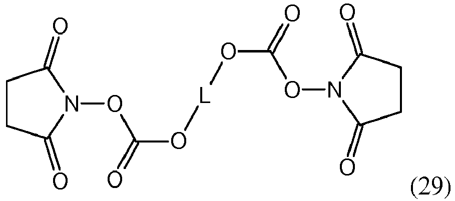 wherein M', M and L are as defined elsewhere in this specification, in particular herein above, R₁ is as defined with respect to formula (5), preferably C1-C5 alkyl. Preferably, in formula (29), L is a moiety of formula (25) as defined elsewhere in this specification. Preferably, in formula (28), M is a moiety of formula (20).

In an embodiment, said second monomers (type B or linker monomer) are selected from compounds of formula (31) and (32): 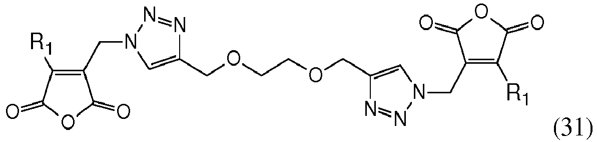 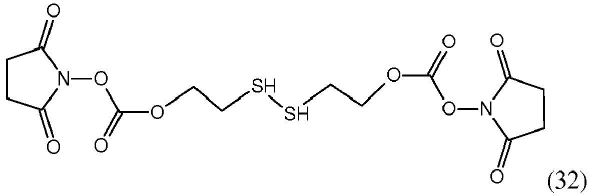 wherein R₁ is as defined with respect to formula (5), preferably a C1-C5 alkyl.

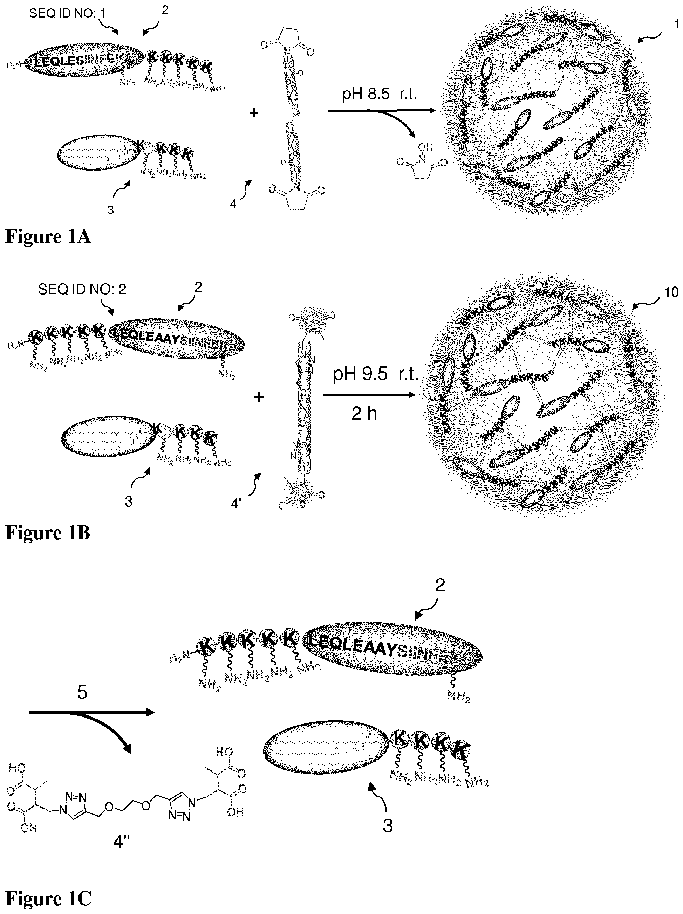

The reaction of the method of the invention may take place in aqueous solution at basic pH (e.g. 8-10) and RT (25°C), see e.g. Figs 1A and 1B. The linker monomer is preferably added from DMSO solution, which avoids hydrolysis of the monomer. During the polymer forming reaction, the aqueous solution containing the reactants is preferably shaken.

The method of the invention can be conducted while using one type A monomer, selected from A₁ as defined in this specification, but also with two different type A monomers, such as A₁, A₂ and generally A_{X}. The invention is not limited with respect to the number of different type A monomers, but can be conducted with 1, 2, 3, 4, (A₁, A₂, A₃, A₄), up to 5, up to 10, up to 50 or even more different type A monomers (see definition of y), wherein these monomers may be selected independently from monomers as defined for A₁ and/or A₂, for example bioactive peptides and molecules comprising an adjuvant.

In some aspects and embodiments, the invention provides the use of the linker monomers, for example of formula (2), (28), (29), (31), (32), and more generally linker monomers comprising functional groups of formula (5), (5') or (6) and preferably linker moieties L of formula (10) or (25), as defined herein above, for cross-linking bioactive compound comprising free amino groups.

In some aspects and embodiments, linker monomers of formula (2), (28), (31), and more generally linker monomers comprising functional groups of formula (5) or (5') and preferably linker moieties L of formula (10), and molecules comprising one or more free amino groups (such as said type A monomers) for producing polymers that decompose in response to a particular pH change or value, preferably under acidic pH, as further described in more detail elsewhere in this specification.

The invention also concerns polymers obtained by the methods of the invention.

The polymer of the invention preferably constitutes and/or provides a platform or delivery system, which may be used for delivering different molecules, such as peptides and others.

In an embodiment, the polymers of the invention are selected from pH responsive polymers and redox-responsive polymers. Accordingly, the polymers are designed to release bioactive moieties A₁ and/or A_{X} in dependence of the redox-environment and/or in dependence of pH.

The release conditions are defined by the particular type B or linker monomer used for conducting the method of the invention. If a linker monomer with D corresponding to formula (5) or (5') is used (e.g. linker monomers of formulae (28) or (31)), a polymer is obtained that releases the bioactive molecules under acidic conditions. Preferably, in this case L is a moiety of formula (10).

If a linker monomer is used with D corresponding to formula (6) and L comprising a disulphide bond (e.g. corresponding to formula (25)), as is the case e.g. with linker monomers of formula (29) and (32), a polymer is obtained that releases the bioactive molecules under reducing conditions.

Preferably, said bioactive molecules A₁ and/or A_{X}, etc. are released without leaving any undesired trace (tracelessly) on said bioactive molecules, in particular under the predefined conditions (reducing conditions, acidic conditions). For example, the bioactive peptide's amine groups, which were used to connect with the linker monomer, are completely recovered upon release.

Furthermore, when the polymers decompose, there is no empty delivery matrix, particle shell and the like. Preferably, the products of the decomposition, besides said bioactive molecules A₁ and/or A_{X}, are not toxic readily metabolized by the organism to which the polymers were administered.

In an embodiment, said linker L is adapted to react under conditions selected from (i) acidic conditions and/or (ii) reducing conditions so as to disconnect and release said molecules A₁, A2, and generally A_{X}, when the polymer obtained by the method of the invention, for example the polymer comprising a structure of formula (40) or (40a), is exposed to said conditions (i) and/or (i).

In some embodiments, the polymers release bioactive molecules (A₁, A₂, A_{X}) under reducing conditions. For example, reducing conditions are conditions that are equivalent to the conditions obtained in the presence of 2 mM reducing agent DTT. In the absence of such reducing conditions, in particular under physiological conditions, the polymer remains preferably intact.

In an alternative embodiment, reducing conditions are conditions obtained in the presence of 0.5 mM reducing agent glutathione (GSH), preferably 1 mM GSH, more preferably 1.5 mM GSH, even more preferably 2 mM GSH, most preferably 2.5 mM GSH, for example 3 mM GSH. Preferably, in the absence of such reducing conditions, in particular under physiological conditions, the polymer remains preferably intact.

In some embodiments, the polymers release bioactive molecules (A₁, A₂, etc.) under acidic conditions, preferably at a pH of < 7, preferably ≤ 6.5, even more preferably ≤ 6.0, most preferably ≤ 5. In an embodiment, polymers release bioactive molecules at a pH in the range of 6.3-6.9, more preferably 6.4-6.8. At a pH above any one of these values, depending on the embodiment, and in particular under physiological conditions, the polymer remains preferably intact.

In an embodiment, the polymer of the invention is present in form of nanoparticles having an average hydrodynamic diameter of <500nm, preferably <200 nm, and more preferably <100 nm.

In an embodiment, the polymer of the invention has a molecular weight of larger than 5 kD and smaller than 1000kD, preferably larger than 15 kD and smaller than 200 kD. The molecular weight may be estimated by comparing the HPLC-SEC curves with those of other proteins, for example with that of antibodies.

In some embodiments, the polymer obtained by the present invention comprises one or several moieties of formula (40) and/or (40a): 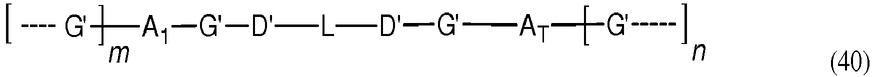 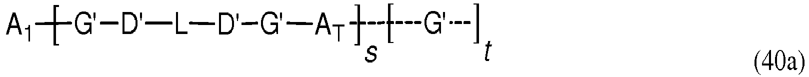
wherein A₁ and L are as defined as with respect to the monomers used for the method of the invention, including preferred embodiments thereof;
m, n and t are independently 0, 1 or >1, s=m+1;
T is 1 or 2, wherein if T is 2, A_{T} is A₂, which is different from A₁;
A₂ is defined as A_{X} in formula (1'), with y=1 and X=2.
L is an organic moiety comprising from 1 to 1000 carbons and from 0 to 300 heteroatoms;
G' are preferably the derivatized forms of G. In a preferred embodiment, G is a free amine group (or primary amine, -NH₂), in which case G' is thus preferably the derivatized amine group, that has reacted with the linker monomer to form the polymers of preferred embodiments of the invention. Generally, G' is -NH- in an amide moiety.

D' is preferably a moiety the structure of which determines, optionally together with the structure of L and with moiety G', the release characteristics of the polymer (e.g. pH or redox sensitive). D' preferably has 1-50 carbons and 1-20 heteroatoms, preferably 1-20 carbons and 1-10 heteroatoms, most preferably 1-10 carbons and 1-5 heteroatoms. Said heteroatoms are preferably selected from O, S and N, more preferably from A and S, most preferably from O.

The moieties -G'-D'- and -D'-G'- are moieties obtained by the reaction of groups D with groups G as defined with respect to the monomers used for the method of the invention, and m and n (or s and t, as applicable), are independently integers that represent the numbers of groups G of A₁ and A₂ that have reacted with groups D of the second monomer of formula (2), wherein m, n, s, and t are independently equal or smaller than q and r as defined with respect to formula (1) and (1'), preferably 1-200, more preferably 2-100, most preferably 3-50.

As expressed by m and n (or s and t) and the dotted lines, the moieties A₁ and A_{T} are preferably connected via further linkers L to further moieties A₁ and/or A_{T}. Assuming that A_{T} is A₂ and thus a moiety that is different from A₁ in formula (40) and (40a), the overall polymer macromolecule may further comprise elements A₁-L-A₁ and A₂-L-A₂ (the functional groups G' and D' are not shown for facilitating understanding). Furthermore, within the polymer, any given moiety A₁ (or A₂), may have the same or a different value for m (or n), meaning that the number of linkages from a given moiety A₁ (or A₂) to another moiety A₁ (or A₂) is not necessarily constant and may vary within the polymer, as the system is designed to allow for multiple connections of any A₁ (or A₂) to other moieties A₁ and/or A₂, as illustrated with reference numerals 1 and 10 in Figure 1A and 1B, respectively.

In a preferred embodiment, m may be 0, 1 or an integer larger than 1; n is 0, 1 or larger than 1, s is n+1 and t = n. Preferably, m and n are independently, 1 or > 1, preferably 2 or >2, most preferably 3 or >3.

Preferably, m, n, s and t are ≤200, more preferably ≤100, even more preferably ≤50, most preferably ≤20. Generally, s= m+1, and t = n.

Preferably m, n and t are, independently, integers of 1-200, preferably 1-200, more preferably 2-50, and s=m+1.

As the skilled person will understand, the relation of q and r(x) in formula (1) and (1') with m and n in formula (40) or with s and t in formula (40a) is as follows: q and r(x) represent the number of the totally actually available free groups G, whereas m+1 and n+1 may represent, at least in the elements of structure (40), the number of those groups G that have reacted in the final polymer and are thus present in the form of moieties connected to the linker moiety, such moieties -G'-D'- and -G'-D'-, for example. In an embodiment, not all groups G react, such that some free groups G remain in the final polymer. Therefore, m, n, s, and t are generally smaller than q and r.

It is noted the r(x) represent the total numbers of free functional groups G of the corresponding A_{X.} For example, for A₂, r(x) is r(2), which represents the number of free groups of A₂. Since any other A_{X} may be different from A₂, for example A₃ may have r(3) free groups G, wherein r(2) and r(3) may be the same or different.

Preferred embodiments of A₁, A_{T} and L are as defined herein above, in connection with the monomers used in the method of the present invention.

In a preferred embodiment, L is an organic moiety comprising from 1 to 100 carbons and from 0 to 50 heteroatoms.

In an embodiment, the moiety -G'-D'- and/or -D'-G'- comprises one selected from an amide bond and a carbamate ester.

In an embodiment, the moiety -G'-D'- and/or -D'-G'- comprises a moiety of formula (45): 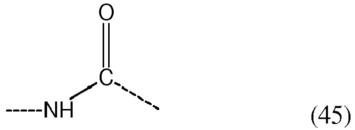 wherein G' is a derivatized amino group (-NH-) in formula (45), and wherein the dotted lines on the left and right of formula (45) represent the bonds by which -G'-D'- and/or -D'-G'- is connected to A₁ or A_{T} and to L, respectively.

In an embodiment, the moiety -G'-D'- and/or -D'-G'- comprises a moiety of formula (46): 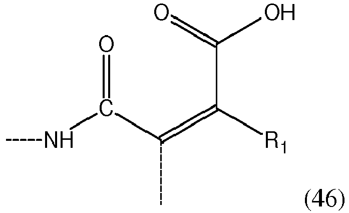
wherein R₁ is as defined herein above with respect to formula (5), preferably a C1-C5 alkyl; G' is a derivatized amino group, and wherein the nitrogen in formula (46) is the nitrogen by which said amino group is connected to the linker L;
and wherein the dotted lines on the left and bottom of formula (46) represent the bonds by which -G'-D'- and/or -D'-G'- is connected to A₁ or A_{X} and to L, respectively.

In an embodiment, the polymer of the invention comprises one or several moieties of formula (47) or (48): 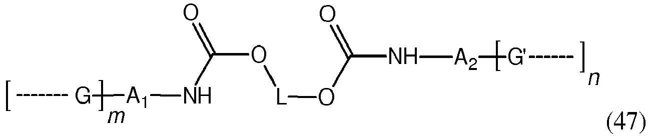 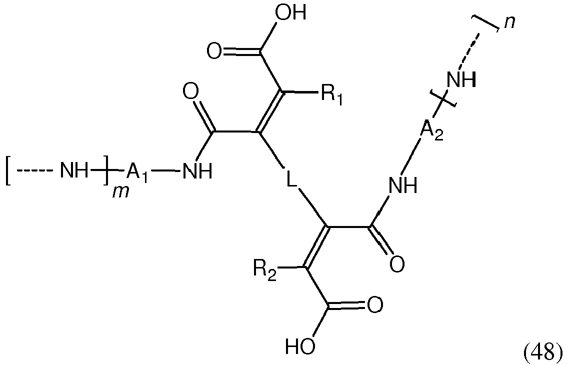
wherein A₁ and A₂, m and n, in as far as present, are as defined in this specification, and,
wherein L is an organic moiety comprising from 1 to 1000 carbons and from 0 to 300 heteroatoms,
R₁ and R₂ may be the same or different and are selected substituents as defined for R₁ with respect to formula (5) above.

Preferred embodiments for L are disclosed with respect to the type B (linker) monomers herein above.

In an embodiment, in particular with respect to formula (47), L comprises a disulphide moiety. In this case, the polymer is preferably redox-responsive as detailed elsewhere in this specification.

Preferably, L is a linear moiety, optionally comprising O and S heteroatoms. In an embodiment, L comprises a linear alkdiyl and/or one or more polyethylene glycol (PEG) moieties.

In an embodiment, the polymer of the invention comprises one or several moieties of formula (49) and/or (50): 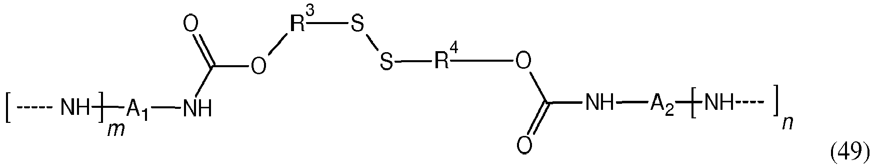 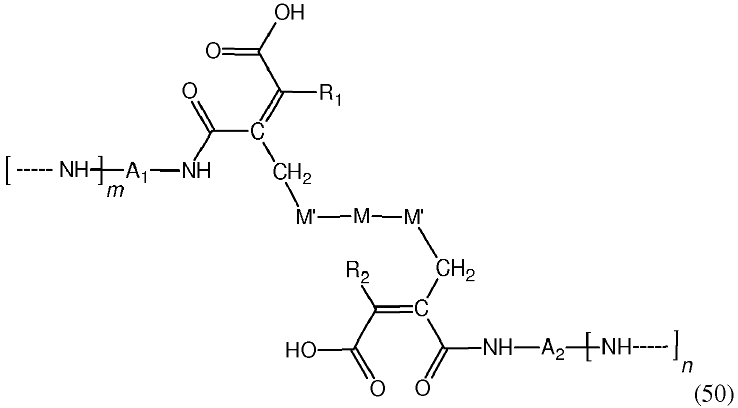
wherein A₁, A₂, m, n are as defined herein above with respect to formula (1) and (1');
wherein m and n are preferably >1 and 100 or less.
   M an organic moiety comprising from 1 to 1000 carbons and from 0 to 300 heteroatoms;
   M' is selected from the moieties of formula (11), (12), (13), (14) and (15) below: 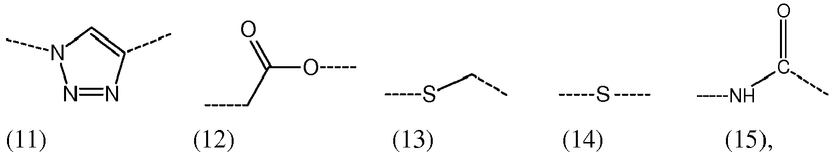
      wherein, in formulae (11) to (15), the dotted line on the right represents the bond to M and the dotted line on the left represents the bond to the -CH₂- moiety in formula (10);
      R₁ and R₂ may be the same or different and are selected from substituents as defined for R₁ and with respect to formula (5). Preferably, R₁ and R₂ are selected from C1-C5 alkyl;
      R³ and R⁴ are preferably selected independently from -(CH₂)v- wherein v is selected from integers of 1-50, preferably 1-10, more preferably 2-5, most preferably v is 2.

Preferably, M is a moiety selected from the moieties of formulae (20)-(23) as defined elsewhere in this specification.

Regarding the conditions under which said polymer disintegrates and releases A₁ and A_{X}, the same as indicated herein above with respect to the monomers applies. For example, with respect to polymer (47), with L being a moiety of formula (15), the polymer with release the type A monomers under reducing conditions. On the other hand, polymer (48), in particular where L is a moiety of formula (10), said polymer will generally release the type A monomers under acidic conditions.

While certain of the preferred embodiments of the present invention have been described and specifically exemplified above, it is not intended that the invention be limited to such embodiments. Various modifications may be made thereto without departing from the scope and spirit of the present invention, as set forth in the following claims.

### Examples

### A. Materials

All the synthetic long peptides (SLPs) were purchased from GenScript (Piscataway, New Jersey, USA). Pam₃CysSer-(Lys)₄ (Pam) was purchased from InvivoGen (San Diego, California, USA). Bis-amine-CpG (5'-/5AmMC6/TCCATGACGTTCCTGACGTT/3AmMO/- 3', (SEQ ID NO: 15) amine-functionalization at 5'- and 3'-positions) was purchased from Integrated DNA Technologies (Coralville, Iowa, USA). Montanide™ ISA 51 VG was a gift from SEPPIC (Paris, France). Murine granulocyte-macrophage colony-stimulating factor (GM-CSF) was purchased from PeproTech (London, UK). Lipopolysaccharide (LPS) and other chemicals were purchased from Sigma-Aldrich (St. Louis, Missouri, USA). Unless otherwise noted, all chemical and biological reagents were used as received.

Pierce™ quantitative fluorometric peptide assay, ProLong™ diamond antifade mountant, Alexa Fluor™ 647 N-hydroxysuccinimide (NHS) ester, LysoTracker™ Red DND-99, Hoechst 33342, CellTrace™ carboxyfluorescein succinimidyl ester (CFSE) cell proliferation kit, and LIVE/DEAD™ fixable violet dead cell stain kit were purchased from Thermo Fisher Scientific (Waltham, Massachusetts, USA). Cytofix/Cytoperm™ fixation/permeabilization kit was purchase from BD Bioscience (San Jose, CA, USA). Mouse CD8⁺ T cell isolation kit was purchased from Miltenyi Biotec (Bergisch Gladbach, Germany).

Antibodies for fluorescence-activated cell sorting (FACS) including anti-CD16/32 (Clone: 93), anti-CD8α (Clone: 53-6.7), anti-CD11c (Clone: N418), anti-CD40 (Clone: 3/23), anti-CD44 (Clone: IM7), anti-CD62L (Clone: MEL-14), anti-CD80 (Clone: 16-10A1), anti-CD86 (Clone: GL-1), and anti-IFN-γ (Clone: XMG1.2) were purchased from BioLegend (San Diego, California, USA). iTAg Tetramer/PE-H-2 Kb OVA (SIINFEKL) was purchased from MBL (Woburn, Massachusetts, USA).

### B. Instruments

Nuclear magnetic resonance (NMR) spectra were acquired on a Bruker AVANCE NEO 400 MHz spectrometer (Billerica, Massachusetts, USA). Electrospray ionisation mass spectra (ESI-MS) was acquired on a LTQ Orbitrap ELITE ETD (Thermo Fisher Scientific). Matrix-assisted laser desorption/ionization time-of-flight mass spectra (MALDI-TOF-MS) was aquired on an Autoflex Speed (Bruker, Billerica, Massachusetts, USA). Polycondensate neoepitope (PNE) was characterized by PL-gel permeation chromatography (GPC) 50+ Integrated GPC/SEC System (Agilent, Santa Clara, California, USA) and UltiMate™ 3000 ultra high-performance liquid chromatography (UHPLC) system (Thermo Fisher Scientific) equipped with a Hypersil Gold™ C18 selectivity LC column or a BioBasic™ SEC 300 LC column, and detectors of diode array detector (UV, DIONEX UltiMate™ 3000) and charged aerosol detector (CAD, DIONEX Corona ultra RS). The size of particle was measured by dynamic light scattering (DLS) on Malvern NanoZS (Worcester, UK). The atomic force microscopy (AFM) images of PNEs were acquired on a Park NX-10 (Suwon, South Korea) with non-contact amplitude modulation (PPP-NCHR, Park system) in ambient condition. The fluorescence intensity of samples was measured with a Varioskan® Lux microplate reader (Thermo Fisher Scientific). All the flow cytometry data were acquired using an Attune NxT flow cytometer (Thermo Fisher Scientific). Confocal fluorescent microscope images were acquired with a LSM 700 with 40X or 63X oil objectives (Zeiss, Oberkochen, Germany). Mouse tissue imaging was conducted with an *in vivo* imaging system (IVIS, PerkinElmer, Waltham, Massachusetts, USA).

### C. Mice and cells

Experimental procedures in mouse studies were approved by the Swiss authorities (Canton of Vaud, animal protocol ID 3206) and performed in accordance with École Polytechnique Fédérale de Lausanne (EPFL) CPG guidelines. Six- to eight-week-old female Thy1.2⁺ C57BL/6 mice, TCR-transgenic OT-I mice (B6-Tg(TcraTcrb)1100Mjb/J) were purchased from The Jackson Laboratory (Bar Harbor, Maine, USA) or Charles River Laboratories (Lyon, France) and maintained in the animal facility. B16F10 murine melanoma cell line was purchased from ATCC (Manassas, Virginia, USA) and cultured in DMEM complete medium supplemented by fetal bovine serum (FBS, 10%), Penicillin (100 UmL⁻¹), Streptomycin (100 µgmL⁻¹). Immature bone marrow-derived dendritic cells (BMDCs) were isolated from C57BL/6 mice (Charles River laboratory, Wilmington, Massachusetts, USA), and cultured in RPMI 1640 complete medium containing HI-FBS (10%), L-glutamine (2 mM), Penicillin (100 UmL⁻¹), Streptomycin (100 µgmL⁻¹), and 2-mercaptoethanol (β-ME, 50 µM) with GM-CSF (20 ngmL⁻¹) for 6 days at 37 °C with CO₂ (5%) before use. Naive OT-I CD8⁺ T cells were isolated from splenocytes of OT-I mice (Jackson laboratory) with a mouse CD8⁺ T cell isolation kit.

### D. Synthesis of redox sensitive linker (bis-functional Monomer B_{red}, Mon_{red})

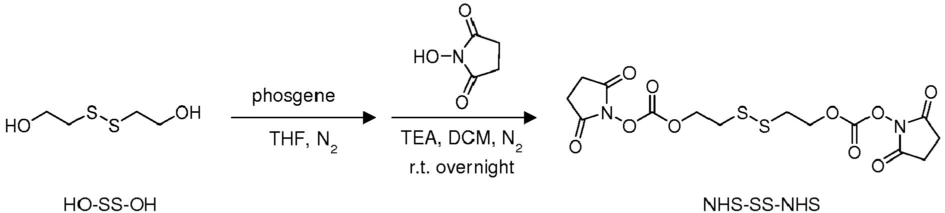

2-Hydroxyethyl disulphide (1.329 g, 8.616 mmol) was dissolved in anhydrous tetrahydrofuran (THF, 20 mL) in a 100-mL round-bottom flask, followed by addition of phosgene solution (12.5 mL, 15% w/w, 18.95 mmol) in anhydrous THF (10 mL). The reaction mixture was stirred under room temperature (rt) for 2 hours under the protection of N₂, and then concentrated under vacuum. The remaining residue was dissolved again with anhydrous dichloromethane (DCM, 10 mL) and mixed with *N*-hydroxysuccinimide (NHS, 2.182 g, 18.95 mmol) and anhydrous triethylamine (TEA, 1.918 g, 18.95 mmol) in DCM solution (45 mL). The reaction was stirred at rt overnight under the protection of N₂. The solvent was removed by rotary evaporator. The crude product was purified with silica chromatography (DCM:Methanol=10:1) and recrystallized with icy petroleum. The acicular crystal (2.14 g, yield: 57%) was dried under vacuum and characterized by ¹H NMR and ESI-MS. (¹H NMR (400 MHz, chloroform-*d*, 25 °C, TMS): δ=4.61 (t, ³*J*(H, H)=4 Hz, 4H, CH₂), 3.08 (t, ³*J*(H,H)=4 Hz, 4H, CH₂), 2.88 (s, 8H, CH₂). ESI (m/z): [M+Na] ⁺=459.0).

### E. Synthesis of the pH sensitive linker (Mon_{pH})

### 1. Preparation of 2-(bromomethyl)-3-methyl maleic anhydrate

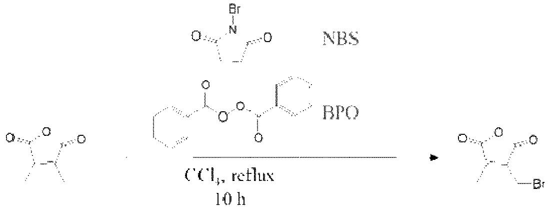

A mixture of dimethylmaleic anhydride (5.04 g, 50 mmol), NBS (14.24 g, 100 mmol), and a catalytic amount of benzoyl peroxide (200 mg, 0.83 mmol) in carbon tetrachloride (300 mL) was gently refluxed for 5 h in a 500 mL round-bottom flask. The reaction mixture was allowed to cool to room temperature, a second portion of benzoyl peroxide (200 mg, 0.83 mmol) was added, and again the refluxing was continued 5 h longer. The mixture was left overnight at room temperature and then filtered. The residue was washed with CCl4 (25 mL × 2); the combined organic layer was washed with water (100 mL × 2) and brine (100 mL), and then dried over Na2SO4 and concentrated in vacuo to furnish thick yellow oil, which was purified by chromatography on a silica gel column [elution with petroleum ether/ethyl acetate (8: 2)] to obtain a crude product (7.0 g) and then further purified by distillation using Kugelrohr apparatus. The second fraction obtained at 90 °C was the aimed product.

(Reference: J. Org. Chem. 1998, 63, 9557-9558). 1H NMR: (CDCl3, 200 MHz) δ 2.18 (s, 3H), 4.20 (s, 2H).

### 2. Preparation of 2-(azidomethyl)-3-methyl maleic anhydrate

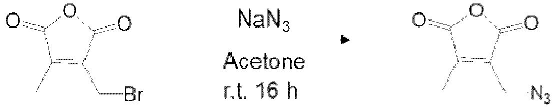

2-(azidomethyl)-3-methyl maleic anhydrate (310.5 mg, 1.5 mmol) was dissolved in acetone (10 ml). Sodium azide (97.5 mg, 1.5mmol) was added in one portion. The suspension was stirred over night at room temperature. After filtering the solvent was evaporated. The remaining oil was dissolved in ethyl acetate (20 ml) and washed with water (20 ml). Afterwards the organic layer was washed with 20 ml brine and dried over Na2SO4. Concentration in vacuo, followed by chromatographic purification over a silica gel column using hexane/ethyl acetate (7:3) as mobile phase gave pure product. (Reference: J. Am. Chem. Soc. 2012, 134, 10169-10173). NMR spectra: (400 MHz ,CDCl3) 4.29 (q, J = 1.01 Hz, 2H), 2.17 (t, J = 1.01 Hz 3H))

### 3. Click reaction (pH responsive linker)

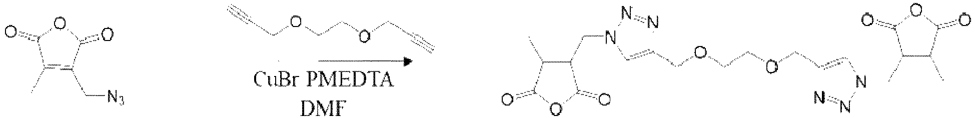

2-(azidomethyl)-3-methyl maleic anhydrate (150 mg, 0.89 mmol, 2.0 equiv.) and ethylene glycol 1,2-bis(2-propynyl) ether (62 mg, 0.45 mmol, 1.0 equiv.) were dissolved in THF, and bubbled with nitrogen for 15 min to get rid of oxygen. CuBr was added and the suspension was bubbled with nitrogen for another 15 min. PMDETA was then added with a microsyringe, and stirred in dark at 40 °C for 4 h. The reaction mixture was concentrated and purified with silica column (pure Ethyl Acetate). 1H NMR spectra: (CDCl3, 200 MHz) δ 7.78 (s, 2H, triazole), 5.41 (s, 4H, =C-CH2-N=); 4.73 (s, 4H, =C-CH2-O-); 3.75 (s, 4H, -O-CH2-CH2-O-); 2.18 (s, 6H, CH3-).

**F. PNE of SLP only (PNE(LEQ)) using the redox-sensitive linker:** LEQLESIINFEKLKs (LEQ, 50 µg, 22.55 nmol) in phosphate-buffered saline (PBS, pH 8.5, 5 µL) was diluted with PBS (3.77 µL, pH 8.5), and then mixed with Mon_{red} (112.75 nmol) in anhydrous dimethyl sulfoxide (DMSO, 1.23 µL). The mixture was shaken with an Eppendorf ThermoMixer (Eppendorf, Hamburg, Germany) at 25 °C, 600 rpm, for 30 min. The resultant product was purified by PBS (pH 7.4, 200 µL × 3) with Amicon® centrifugal filters (Merck Millipore, Burlington, Massachusetts, USA) with molecular weight cut-off (MWCO) of 3 kDa, to get rid of unreacted peptides or Mon_{red}. PNE(LEQ) was characterized with DLS, ¹H NMR, AFM and HPLC.

**G. PNE of SLP and adjuvant using the redox-sensitive linker:** LEQ (50 µg, 22.55 nmol) in PBS (pH 8.5, 5 µL) was mixed with the solution of Pam (8.5 µg, 5.6 nmol) in physiological water (1.7 µL), and further diluted with PBS (2.07 µL, pH 8.5). Mon_{red} (40 mgmL⁻¹ in anhydrous DMSO, 1.23 µL, 112.75 nmol) was then added. The mixture was shaken in an Eppendorf ThermoMixer at 25 °C, 600 rpm for 30 min, and purified by PBS (pH 7.4, 200 µL × 3) with Amicon® centrifugal filters (MWCO 3 kDa) to get rid of unreacted peptides, adjuvants and Mon_{red} to afford PNE(LEQ-Pam). The resultant product was characterized with DLS, GPC, HPLC and AFM **(****Figure 1**). Similar synthesis procedure was performed to prepare other PNEs with various epitopes and adjuvants. The mole ratio of SLP, adjuvant, and Monred was optimized for each PNE.

**H. Preparation of non-degradable PNE (non-deg. PNE):** Non-degradable PNE(LEQ-Pam) was prepared similarly as described above by replacing Mon_{red} with a non-degradable monomer B, bis(sulfosuccinimidyl)suberate (Mon_{BS3}, 112.75 nmol), in anhydrous DMSO (1.61 µL). The other procedures were the same as PNE(LEQ-Pam).

### I. PNE of SLP only (PNE(LEQ)) using the pH-sensitive linker

LEQLESIINFEKLK₅ (LEQ, 50 µg, 22.55 nmol) in phosphate-buffered saline (PBS, pH 9.5, 5 µL) was diluted with PBS (2.34 µL, pH 9.5), and then mixed with Mon_{pH} (112.7 mmol, 50 equiv.) in anhydrous dimethyl sulfoxide (200 mg/mL in DMSO, 2.66 µL). The mixture was shaken with an Eppendorf ThermoMixer (Eppendorf, Hamburg, Germany) at 20 °C, 800 rpm, for 4h. PNE_{pH}(LEQ) was characterized with DLS, and HPLC.

Different PNEs according to Examples 1-13 below were synthesised using the same reaction principles as set out in F. and I.

### J. Characterizations of PNEs

**NMR:** Free LEQ (200 µg) and PNE(LEQ) (200 µg) were ultrafiltered with D₂O (200 µL × 3) using Amicon® centrifugal filters (MWCO 3 kDa, 10000 rpm) to get rid of H₂O, DMSO, salts, and other possible impurities. The trace of water and protons from acid phosphate was further removed by repeating lyophilizing and dissolving with D₂O. The purified free LEQ and PNE(LEQ) were re-dissolved in D₂O (400 µL) respectively, and measured by ¹H NMR spectrometer.

**UHPLC:** LEQ, Pam, and PNE(LEQ-Pam) each was diluted in PBS (pH 7.4) with a concentration of 0.1 mgmL⁻¹. A mobile phase gradient (A=acetonitrile, B=H₂O+0.1% trifluoroacetic acid (TFA), B at 90% for 0-1 min, 90-10% for 1-18 min, 10-90% for 18-19 min, and 90% for 19-20 min at flow rate of 0.4 mLmin⁻¹) was used on a Hypersil Gold™ C18 selectivity LC column at 40 °C. The loading efficiency of LEQ in PNE(LEQ-Pam) was calculated from the integral area of CAD curves. To verify the increased MW of PNE(LEQ) and PNE(LEQ-Pam) comparing with free PNE, a mobile phase of NaCl solution (200 mM, pH 5.75 at flow rate of 1.0 mLmin⁻¹) was used on BioBasic™ SEC 300 LC column.

**GPC:** LEQ and PNE(LEQ-Pam) in PBS (0.1 mgmL⁻¹, pH 7.4) were injected into GPC (100 µL each sample injection) and analyzed with a mobile phase of NaCl (100 mM) and TFA (1%) and flow rate of 1.0 mLmin⁻¹.

**DLS:** PNEs were diluted with PBS (0.1 mgmL⁻¹, pH 7.4) in Fisherbrand™ PS semi micro cuvettes at rt for DLS measurement with Marven NanoZS. Data represented 3 independent samples for each PNE.

**AFM:** Freshly cleaved mica was functionalized by an aliquot of (3-aminopropyl) triethoxysilane (APTES) solution (1%) for 2 min at rt, rinsed by mili-Q water, and dried by air flow. The sample solutions of LEQ, Pam, PNE(LEQ), and PNE(LEQ-Pam) (10 µL each) were dispersed on functionalized mica followed by rinsing with mili-Q water 4 min later. The mica with samples was then dried with mild N₂ gas flow and ready for AFM imaging. The AFM images with a resolution of 512×512 pixels were obtained using non-contact cantilevers with a resonance frequency of 330 kHz and a force constant of 42 Nm⁻¹. To precisely control the cantilever in each measurement, a weak tip-sample interaction was carefully achieved by monitoring a low AFM phase image in the range of ±5°. AFM images were processed by XEI software.

### Release kinetics and characterization of LEQ from PNE(LEQ-Pam)

Freshly prepared PNE(LEQ-Pam) (50 µg) diluted in PBS (0.1 mgmL⁻¹, 490 µL, pH 7.4), was added with DL-dithiothreitol (DTT) (10 µL, 100 mM in PBS, pH 7.4). The mixture was aliquot and incubated at rt or 37 °C. At set time points (0, 2, 4, 8, 12, and 24 hours), the aliquot (10 µL) was collected and analyzed by Pierce™ quantitative fluorometric peptide assay according to the kit protocol. The mixture was kept in dark for 10 min and the fluorescent intensity was quantified with a microplate reader (Ex 390 nm, Em 475 nm). The released LEQ was also characterized with MALDI-TOF-MS to measure the molecular weight (MW) and compare with original LEQ.

### K. Fluorescent labelling of LEQ, Pam and PNE(LEQ-Pam)

Solutions of LEQ, Pam, or the mixture of two were added with Alexa Fluor™ 647 NHS ester (10 mgmL⁻¹ in anhydrous DMSO) with equal stoichiometry and then shaken with an Eppendorf ThermoMixer at 25 °C (600 rpm, 10 min). The "labeled" mixture was used for the next step without purification. Additional LEQ, Pam, or both (unlabeled) were then added the labeled mixture followed by the similar procedures for preparation and purification of PNEs as described above. For *in vitro* studies, 10% of LEQ or Pam was fluorescently labeled; for *in vivo* studies, 50% was labeled.

### L. In vivo lymph node targeting and dendritic cell internalization

Fluorescently labeled LEQ, Pam, or PNE(LEQ-Pam) in PBS solution (50 µL, pH 7.4) containing equivalent LEQ (4.51 nmol) and/or Pam (1.12 nmol) were subcutaneously injected into tail base (25 µL for each side, both sides) of C57BL/6 mice (female, 7 weeks old, n=3 per group). PBS (50 µL, pH 7.4) was used as a negative control. The mixture of equivalent LEQ and Pam (25 µL) was pipetted vigorously with Montanide ISA 51 VG (25 µL) to generate a stable emulsion for injection. The mice were sacrificed 24 hours later and inguinal lymph nodes (LNs) were harvested and imaged with IVIS to measure the total fluorescent intensity in LNs (Ex 640 nm, Em 680 nm, exposure time 0.5 s).

The LNs were then ground through a 70-µm cell strainer and the collected cells were washed with FACS buffer (0.2% BSA in PBS, 200 µL × 2). The cells were blocked with anti-CD16/32 at 4 °C for 15 min, and then stained with anti-CD11c (PE/Dazzle™ 594) at 4 °C for another 20 min followed by washing with FACS buffer (200 µL × 3). The stained cells were resuspended in a 4,6-diamidino-2-phenylindole (DAPI) solution (0.1 µgmL⁻¹, 200 µL) and analyzed with flow cytometry.

### M. In vitro BMDC internalization

Immature BMDCs were prepared as described above. On Day 6, the BMDCs were plated in 24-well plates (5×10⁵ cells per well) with RPMI 1640 medium (1 mL, FBS free, penicillin/streptomycin free) and incubated with fluorescently labeled PNE(LEQ-Pam) or other control formulations containing equivalent LEQ (2.25 nmol) and/or Pam (0.56 nmol) at 37 °C with CO₂ (5%). After 6-hour incubation, BMDCs were harvested, washed with FACS buffer (200 µL × 2), and incubated with anti-CD 16/32 at 4 °C for 15 min. The BMDCs were then stained with PE/Dazzle 594-anti-CD11c at 4 °C for 20 min, washed with FACS buffer (200 µL × 2), and resuspended in a DAPI solution (0.1 µgmL⁻¹, 200 µL) for flow cytometry analyses.

### N. In vitro BMDC activation

Immature BMDCs were prepared as described above. On Day 6, immature BMDCs were plated in 24-well plates (5 × 10⁵ cells per well) with RPMI 1640 complete medium (1 mL) supplemented with GM-CSF (20 ngmL⁻¹) and incubated with PNE(LEQ-Pam) or other control formulations containing equivalent LEQ (2.25 nmol) and/or Pam (0.56 nmol) at 37 °C with CO₂ (5%). After 48-hour incubation, BMDCs were harvested, washed with FACS buffer (200 µL × 2), and incubated with anti-CD 16/32 at 4 °C for 15 min. The cells were then stained with a mixture of antibodies of PE-anti-CD11c, PE-Cy7-anti-CD80, BV510-anti-CD86, PerCP-Cy5.5-anti-CD40 at 4 °C for 20 min. The cells were then washed with FACS buffer (200 µL × 2) and resuspended in a DAPI solution (0.1 µgmL⁻¹, 200 µL) for flow cytometry analyses.

### O. In vitro cross-priming of OT-I CD8⁺ T cells

Immature BMDCs were prepared as described above. On Day 6, immature BMDCs were plated in 24-well plates (5 × 10⁵ cells per well) with RPMI 1640 complete medium (1 mL) supplemented with GM-CSF (20 ngmL⁻¹). PNE(LEQ-Pam) and other control formulations containing equivalent LEQ (2.25 nmol) and/or Pam (0.56 nmol) were then added to pulse the BMDCs. SIINFEKL (SIIN, 2.25 nmol) with or without Pam (0.56 nmol) were also added as control samples. The BMDCs were cultured at 37 °C with CO₂ (5%) for 24 hours. The pulsed BMDCs (2 × 10⁴) were first washed with PBS (200 µL × 3) and then co-cultured with naive OT-I CD8⁺ T cells (1 × 10⁵) labelled with CFSE (1 µM for 10 million cells in PBS, 5 min, 37 °C) in complete RPMI 1640 medium (200 µL) at 37 °C with CO₂ (5%). The RPMI complete medium was supplemented with FBS (10%), L-glutamine (2 mM), Penicillin (100 UmL⁻¹), Streptomycin (100 µgmL⁻¹), β-ME (50 µM), sodium pyruvate (1 mM), and HEPES (0.02 M). After a 72-hour co-incubation, the cells were harvested, washed with FACS buffer (200 µL × 2), and incubated with anti-CD16/32 at 4 °C for 15 min. The cells were then stained with PE/Dazzle 594-anti-CD8α at 4 °C for 20 min, washed with FACS buffer (200 µL × 2), and processed similarly for flow cytometry analyses.

### P. Confocal fluorescent microscope imaging

PNE(LEQ-Pam) or the simple mixture containing equivalent LEQ (4.51 nmol) and Pam (1.12 nmol) in PBS solution (5 µL, pH 7.4) were incubated with immature BMDCs (1 × 10⁶) in complete RPMI 1640 culture medium (1 mL) supplemented with GM-CSF (20 ngmL⁻¹). At set time points (6, 24, and 48 hours), the BMDCs were washed with PBS (1 mL × 2) and then stained with LysoTracker™ Red DND-99 (125 nM) for endolysosome staining and Hoechst 33342 (4 µM) for nuclei staining in phenol/serum-free RPMI 1640 medium at 37 °C with CO₂ (5%) for 1.5 hours followed by PBS washing (1 mL × 2). BMDCs were then fixed with 4% paraformaldehyde (PFA, 100 µL) for 10 min, washed with PBS (1 mL × 2), and resuspended in ProLong™ diamond antifade mountant (10 µL). After centrifuging onto a poly-lysine coated glass slide, the BMDCs were imaged with a LSM 700 confocal microscope with a 40X or 63X oil objective.

### Q. In vivo immunization study and tetramer staining

C57BL/6 mice (female, 7-week old) were immunized subcutaneously with LEQ (9.02 nmol) and Pam (2.25 nmol) in a PBS solution (50 µL) in form of a simple mixture, an emulsion formulation in Montainide, or PNE(LEQ-Pam) following a fixed timeline that mice were primed on day 0 and boosted on day 14 and 28. On day 21, 70-µL peripheral blood were collected from the tails of immunized mice, lysated with ACK lysing buffer (1 mL × 2) at rt for 5 min, and then washed with FACS buffer (200 µL × 2). The cells were resuspended in tetramer staining buffer (50 µL, PBS containing BSA (1%), EDTA (5 mM), and dasatinib (50 nM)) with iTAg Tetramer/PE-H-2 K^{b} OVA (SIINFEKL) (1 µL per well) and anti-CD16/32 and incubated at rt for 40 min. Antibodies including FITC-anti-CD8α, PerCP-Cy5.5-anti-CD62L, and BV711-anti-CD44 in a solution of tetramer staining buffer (50 µL) were then added on top of each well and the cells were incubated at 4 °C for another 10 min. The stained cells were washed with DAPI (0.1 µgmL⁻¹, 150 µL × 2) and resuspended in tetramer staining buffer for flow cytometry analyses.

### R. Tumor cell challenging

Immunized mice were challenged with B16F10-OVA tumor cells (3 × 10⁵) 49 days post the prime injection. Tumor growth was monitored every other day from day 11 after tumor inoculation. Tumor area (product of two measured orthogonal diameters) and body weight were measured every 2 days. Mice were euthanized when the body weight loss was >20% of the pre-dosing weight, the tumor area reached 150 mm⁻² (as a pre-determined end point) or the animal had become moribund.

### S. Statistical analysis.

Statistical analysis was performed using GraphPad Prism 8 (GraphPad software, Inc., La Jolla, CA, USA). Unless otherwise noted, the data are presented as mean ± s.e.m. Comparisons of two groups were performed by using two-tailed unpaired Student's t test. Comparisons of multiple groups at a single time point were performed by using one-way analysis of variance (ANOVA). *P*-values were presented as **P* < 0.05; ** *P* < 0.01; *** *P <* 0.001; **** *P* < 0.0001.

### Example 1: Preparation of polycondensates peptides using a peptide monomer and a linker monomer

In these examples, an exemplary neoeptitope is used as a peptides-based monomer in the polycondensates according to an embodiment of the invention. The term polycondensate neoepitopes (PNEs) is used for the polymers according to this embodiment.

A synthetic long peptide (SLP) bearing the neoepitope and multiple amine groups is used as a first monomer (monomer A), as an example of the moiety A₁ in the claims.

The SLP contains SIINFEKL (SII), SEQ ID NO: 3), the CD8 epitope of ovalbumin (OVA), as a model neoantigen (this referred to as LEQ) in the examples section.

As a second monomer, an amine-reactive bi-functional monomer B bearing a disulfide and two N-hydroxysuccinimide (NHS) groups (Mon_{red}) was synthesized for the polycondensation reaction (see D. above), as an example of a moiety of formula (2) in the claims. Monomer A (SLP only) and B in a week basic aqueous solution to prepare the PNE with responsiveness to reduction activity (See F.). The PNE(LEQ) polymer was successfully prepared evidenced by broadened peaks as compared to the peptide monomer in a ¹H NMR spectrum (not shown). PNE(LEQ) exhibited an average hydrodynamic diameter of 8.14 ± 1.54 nm characterized by dynamic light scattering (DLS) and a spherical morphology imaged by atomic force microscopy (AFM).

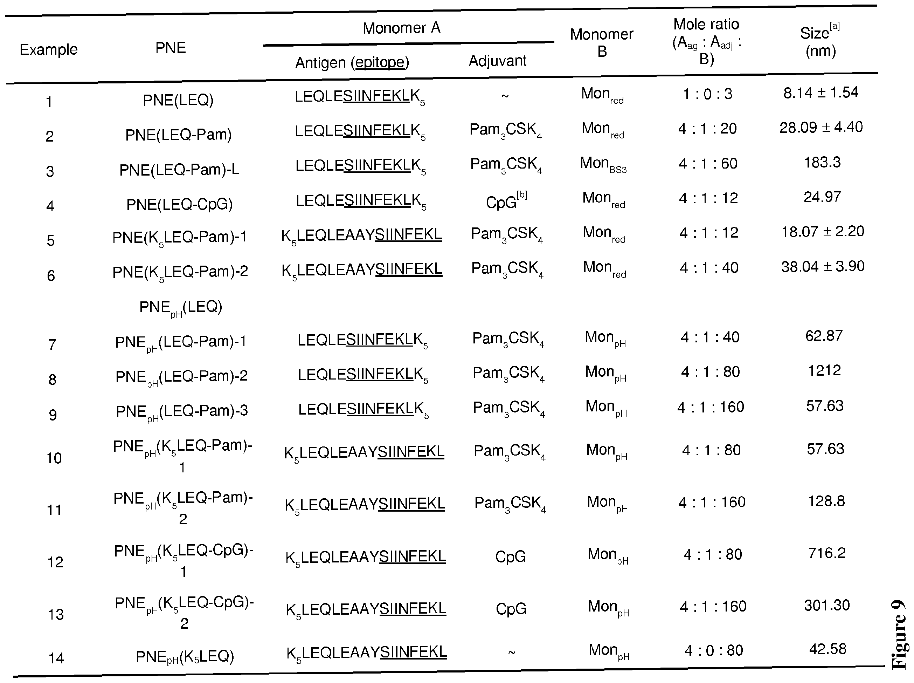

The table in **Figure 9** shows the monomers, the mole rations of the monomers, and the size of the polycondensates of Examples 1-13.

### Example 2: Preparation of polycondensate peptides using a peptide monomer, an adjuvant bearing monomer and a linker monomer

Compared to Example 1, an additional, different monomer A was used, which is Pam₃CSK₄ (Pam), see I. above. Pam is a TLR1/2 agonist functioning as a molecular adjuvant. Pam was selected as it bears multiple amine groups facilitating the direct polycondensation with Mon_{red} and has been shown to potently amplify T cell priming when conjugated with peptides. Using this second monomer A, a self-adjuvanted PNE(LEQ-Pam) was formed with Mon_{red} forming, following the procedure detailed in G. above.

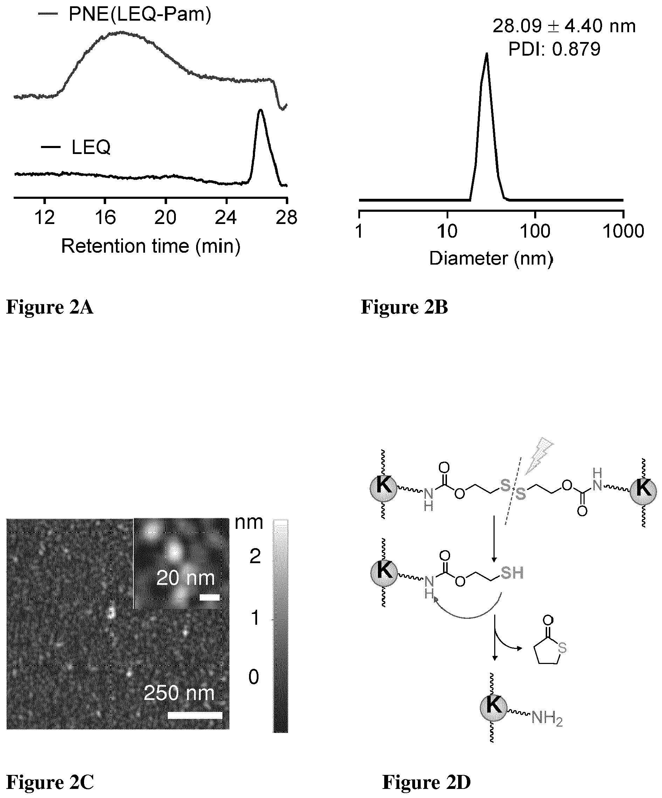

The formation of this polycondensates **1** is illustrated in **Figure 1A**, in which **2** and **3** the neoadjuvant peptide LEQ and pam (monomers A), respectively. The linker (monomer B) is indicated with numeral **4**). PNE(LEQ-Pam) showed increased MW compared to the monomers as observed in both characterizations of gel permeation chromatography (GPC) (**Figure 2A**) and ultrahigh-performance liquid chromatography (UHPLC) equipped with a size-exclusion column (SEC) suggesting the successful copolymerization. Further, negligible amount of both monomers (LEQ and Pam) was detected by HPLC equipped with a C18 column indicating a highly efficient polycondensation reaction. Almost quantitative incorporation efficiency (>99%) and remarkably high loading capacity of cargos (∼46.4% of dry weight was SLP antigen, and 7.9% was Pam adjuvant) has been achieved with the PNE platform. The as-prepared PNE(LEQ-Pam) had a relatively homogeneous size with a mean hydrodynamic diameter of 28.09 ± 4.40 nm (Table in Figure 9, Example 2; Figure 2B, 2C).

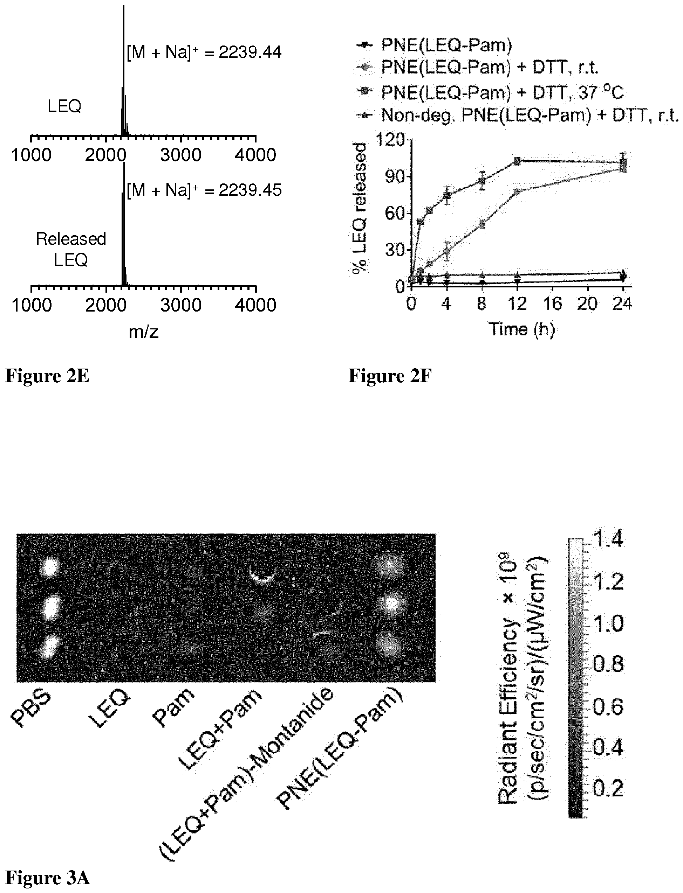

PNE was designed to be degraded in response to intracellular reduction activity facilitating a traceless release of intact peptide antigens through a self-immolative reaction (**Figure 2D**) for unaltered processing and presentation of the designed subunit antigens. Released LEQ from PNE(LEQ-Pam) shared the same MW as the original LEQ showing the evidence of releasing unmodified peptide antigens without residue chemical groups (**Figure 2E**). Consistent with the expectations, reducing agents, such as dithiothreitol (DTT), accelerated the release of LEQ from the PNE at both room temperature (rt) and 37 °C (**Figure 2F**), whereas PNE prepared with a non-degradable monomer B (non-deg. PNE(LEQ-Pam)) (not shown) showed no detectable release of antigens even in the presence of DTT. The intracellular traceless release of antigens could be important to ensure the efficient antigen processing and presentation by DCs.

### Examples 3-14: Preparation of different polycondensate peptides using different peptide and adjuvant containing monomers and different adjuvants

To test the versatility, we have extended the preparation of PNEs to a number of peptide antigens with diverse structure and properties including neoantigens identified from mouse tumors (**Figure 9**). We found the successful formation of PNEs was independent of the sequence or the position of flanking amino acids next to the epitope (**Figure 9**, Examples 5, 6, and 10-14), or the sequence of epitope itself (PNEs of neoantigens identified from MC38 murine colorectal cancer or B16F10 murine melanoma, see **Figure 10**), or the adjuvant molecules, if present (Table 1, Examples 4, 12, 13; triacylated lipopeptide Pam can be replaced by amine-functionalized CpG oligo- or polydeoxynucleotide). PNEs of copolymerized SLP and adjuvant and average sizes ranging from 10.82 to 45.97 nm can be obtained in aqueous solution at rt (25°C) with slightly tuned ratios of monomer A to B (**Figure 9**). The amine group was chosen as the chemical handle for the polycondensation due to the ease of adding flanking lysines without changing the solubility of SLPs. The amine-NHS conjugation based polycondensation was rapid and highly efficient in mild aqueous solutions at rt providing PNE a highly compatible and potentially scalable platform for diverse epitopes.

### Example 15: Targeting efficiency of redox responsive polymers to Lymph nodes (LN)

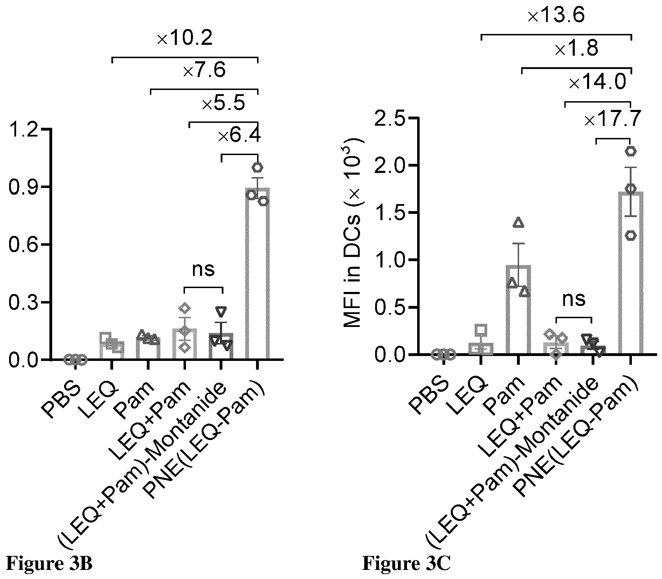

Free SLP, adjuvant, or the mixture of two in the presence or absence of Montanide, or PNE(LEQ-Pam) (Example 2) labeled with fluorescence dye Alexa Fluor™ 647 was injected subcutaneously into C57BL/6 mice at tail base. Twenty-four hours later, the draining LNs were excised for whole-tissue fluorescence imaging and measurement. Monomeric SLP or adjuvant or the simple mixture of two showed limited accumulation in LNs (**Figure 3A**, **3B**). Formulation of LEQ+Pam in Montanide ((LEQ+Pam)-Montanide) did not improve the LN targeting of the vaccine. By contrast, vaccine delivered by PNE exhibited remarkably high LN accumulation reaching a level 10.2-, 7.6-, and 5.5-fold greater than free SLP, adjuvant and the simple mixture of two, respectively. The efficient and fast (within 24 hour) LN targeting
of PNE can be attributed to the well-controlled small size (-30 nm in diameter), which permits the rapid trafficking to lymphoid tissues through afferent lymph.

Next, we assessed the efficiency of antigen capture by the APCs in LNs using flow cytometry. DCs are critical APCs that efficiently process internalized antigens into peptide-MHC complexes (pMHC), which are required for eliciting T cell immune responses. PNE vaccine was captured efficiently by the DCs (CD11c⁺) in LNs with 14.0- and 17.7 fold higher MFI than that of the mixture of free SLP and adjuvant in the absence or presence of Montanide (**Figure 3C**).

### Example 16: The use of the macromolecules for antigen presentation

Next, we examined the impact of PNE platform on antigen presentation.

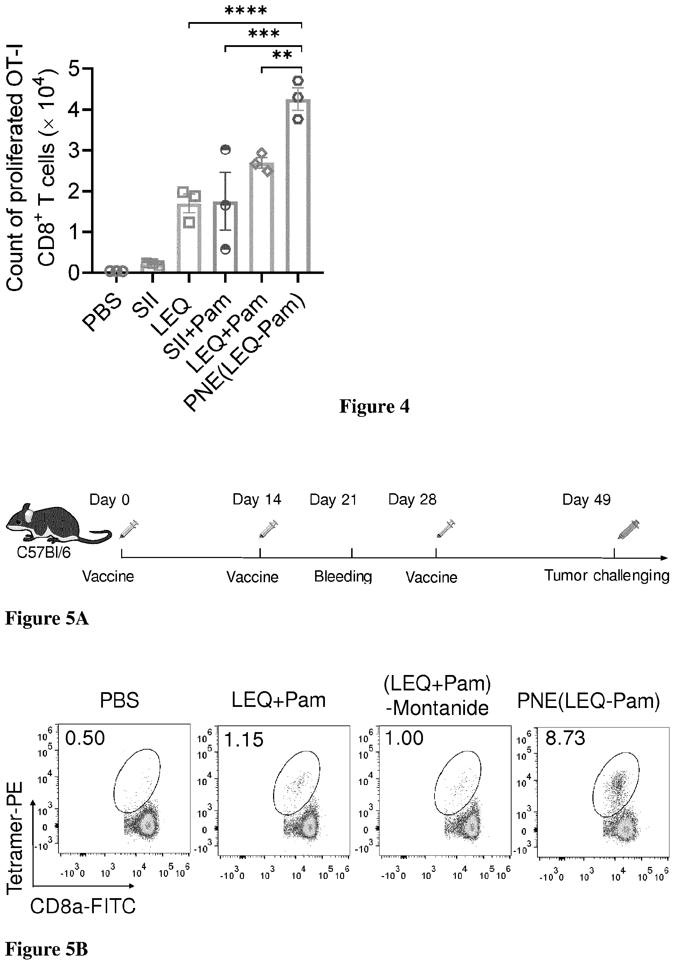

DC maturation is critical for DC functions including antigen presentation and expression of co-stimulatory molecules that are required for T cell stimulation. We collected BMDCs from C57BL/6 mice to assess the capacity of PNEs in converting immature DCs into mature DCs *in vitro* by monitoring the expression level of co-stimulatory markers (CD40 and CD80) with flow cytometry analysis (see C.). PNE with copolymerized Pam promoted the stimulation of BMDCs to the similar level as monomeric Pam or the mixture of free LEQ and Pam. Notably, BMDCs pulsed with PNE(LEQ-Pam) cross-primed the SII-specific naive OT-1 CD8⁺ T cells with a greatly enhanced efficiency compared to monomeric SII or LEQ, or the simple mixture of short or long peptides with Pam, assessed by a 5(6)-carboxyfluorescein diacetate N-succinimidyl ester (CFSE) dilution assay **(****Figure 4**). We found that non-deg. PNE(LEQ-Pam), lacking the redox-responsiveness of the PNE exhibited substantially lower efficiency of cross-priming of OT-1 CD8⁺ T cells (not shown).

### Example 17: Use of polymers of the invention as vaccines

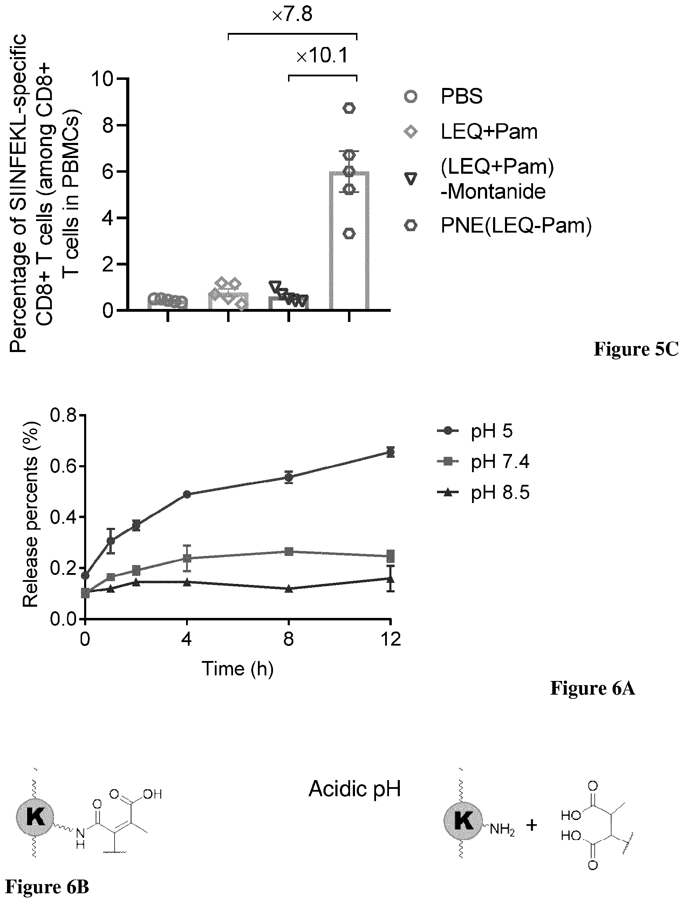

We immunized C57BL/6 mice with PNE(LEQ-Pam) (containing 9.02-nmol LEQ and 2.25-nmol Pam each dose), or the mixture of equivalent doses of LEQ and Pam in form of solution or emulsion in Montanide (**Figure 5A**). Peripheral blood mononuclear cells (PBMCs) were collected after the second immunization and stained with SIINFEKL tetramer to examine the frequency of SIINFEKL-MHC-I tetramer⁺ CD8⁺ T cells (**Figure 4B**). The simple mixture of LEQ and Pam induced minimum T cell immune response with a mean frequency of SIINFEKL-specific CD8⁺ T cells close to the background (0.76% tetramer⁺ among CD8⁺ T cells *vs*. 0.74% in non-immunized mice); the mixture emulsified in Montanide, an oil-based adjuvant used in the clinic for peptide-based vaccines, showed only modest improvement (1.0% tetramer⁺ among CD8⁺ T cells). In contrast, PNE(LEQ-Pam) vaccine elicited 4.2- and 3.2-fold higher frequency of SIINFEKL-specific CD8⁺ T cells compared to the mixture of LEQ and Pam in solution and Montanide, respectively. The majority of the elicited antigen-specific CD8⁺ T cells by PNE(LEQ-Pam) exhibited effector memory phenotype (CD44^{high}CD62^{Llow}) (**Figure 4C**). Notably, PNE(LEQ-Pam) expanded the antigen-specific T_{EM} cells to a remarkably higher number compared to that of the mixture of LEQ and Pam in solution and Montanide (61.1- and 9.7-fold more T_{EM} cells respectively) (not shown). When challenged with B16F10-OVA tumor cells (3 × 10⁵) subcutaneously, mice immunized with PNE(LEQ-Pam) vaccine showed significantly delayed tumor growth (not shown).

### Example 18: Release kinetics of polycondensates based on pH sensitive monomers

**Figure 1B** illustrates the preparation of pH sensitive polymers, in which linker moiety **4'** was used instead of moiety **4** described with respect to Fig. 1A. Reference numeral **10** illustrates the particulate form of the polymers, forming a complex 3D structure.

The pH sensitive PNEs (Examples 7-14) were designed to degrade in response to acidic environment as found, for example, in endosomes of dendritic cells in the lymph node. These PNEs are thus designed to release their antigenic and optionally adjuvant monomers in the dendritic cells.

The polymer PNE_{pH}(K₅LEQ) (Example 14) was exposed to aqueous solutions under various pH values (pH = 5, 7.4, 8.5) as shown in **Figure 6A****.** As expected, release was highest at pH 5).
**Figure 1C** illustrates the release of the bioactive polymer components **2** and **3** as well as the decomposed linker **4".** Reference numeral **5** represents the release conditions, which are her an acidic pH.

### Example 19: Targeting efficiency of pH-responsive polymers to Lymph nodes (LN)

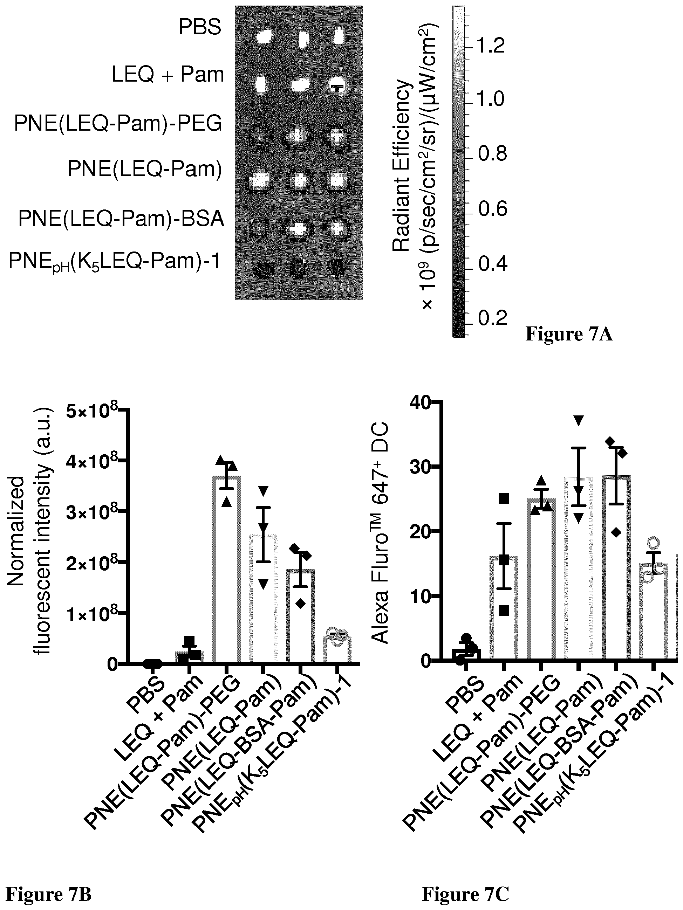

Based on the same protocol as described with respect to Example 15, targeting efficiency to LNs of various compositions, including the pH-responsive polymer PNE_{pH}(K₅LEQ-Pam)-1 (Example 10) was assessed. Treatments include PBS, the co-administration of LEQ and Pam, or the polymer of the invention PNE (LEQ-Pam) alone, together with PEG, or together with BSA, and PNE_{pH}(K₅LEQ-Pam)-1. As shown in **Figures 7A** and **7B**, all polymers according to the invention exhibited better targeting to the LNs, according to the fluorescent imaging.

**Figure 7C** shows that the polymer of the invention showed efficient antigen capture by APCs in LNs, as determined flow cytometry.

### Example 20: Use of PH-responsive polymers of the invention as vaccines

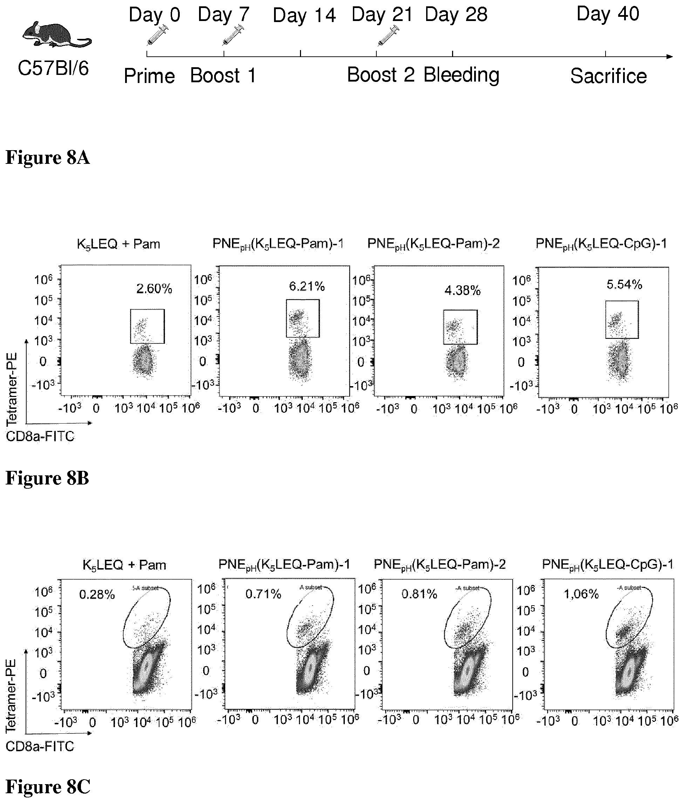

The T cell immune response of PNE vaccines was assessed by flow cytometry based on the experimental setting detailed in Example 17, but with the time line shown in **Figure 8A****.**

**Figure 8B** shows representative flow cytometry plots of SIINFEKL-specific CD8⁺ T cells among all the CD8⁺ T cells in peripheral blood sampled on Day 28, and **Figure 8C** shows representative flow cytometry plots of re-stimulated splenocytes on Day 40. Restimulation was conducted as described with the intracellular staining.

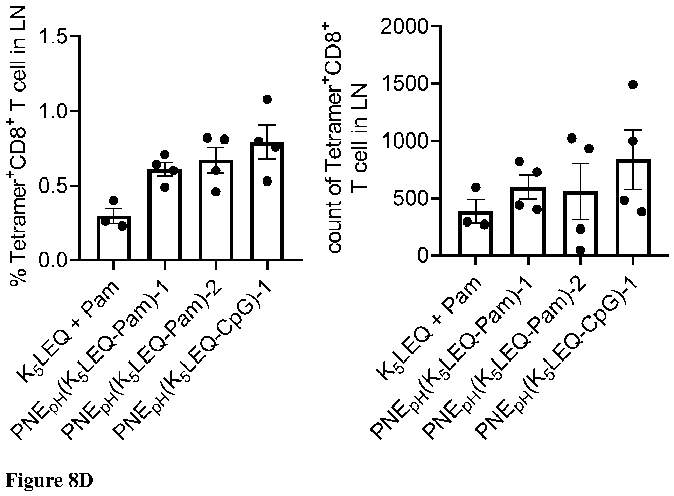

**Figure 8D** shows the Frequency and cell counts of SIINFEKL-specific CD8⁺ T cells among all the CD8⁺ T cells in re-stimulated splenocytes of LN on Day 40.

### Example 21-24: Preparation of different polycondensate peptides using different neoantigens and adjuvant containing monomers and different adjuvants

### Conclusions

We have demonstrated a responsive polycondensate neoepitope as a facile, effective, and versatile vaccine platform for the delivery of peptide neoantigens to enhance personalized cancer immunotherapy. PNE is a highly modular system permitting the co-polymerization of peptide antigens and molecular adjuvants, for example, of diverse structures and properties, which is a highly desired property as individually identified neoantigens from patients could vastly differ in physiochemical properties. We envision that this new strategy can be readily extended to the co-delivery of multiple heterogeneous epitopes in form of SLPs, proteins (e.g., whole tumor cell lysate), or replicon mRNAs/DNAs encoding the neoepitopes. Implementation of various responsive chemistry in the linker-monomer (monomer B) could potentially impart different responsiveness to the PNEs facilitating triggered release of antigens and/or adjuvants by intracellular stimuli including pH change,

Direct assembly of peptide or protein antigens relied on non-covalent assembly or disulfide crosslinking without carriers is actively being pursued as reported in some elegant studies recently. Such "carrier-free" approach has the unique advantage of minimizing the risk of using additional carrier materials whose own immunogenicity and safety profile is still unclear for clinical applications. The PNE approach described here based on a highly efficient covalent conjugation of amines in aqueous solution doesn't require the use of any heating or organic solvent potentially harmful to the biologicals, and therefore showed high promise for the delivery of a wide range of individualized neoepitopes with good compatibility.

## Claims

1. A method of obtaining a polymer, the method comprising reacting at least one monomer selected from monomers (type A monomers) of formula (1):
A₁⁅G]*_{q}* (1)
and optionally y different further monomers of formula (1'),
A_{X}⁅G]ᵣ₍ₓ₎ (1'),
with second monomers of formula (2) (type B or linker monomer)
D-L-D (2),
wherein
A₁ comprises a peptide,
y is 0 (if there is no monomer (1'), 1 or an integer larger than 1;
X is an integer of 2 and up to y+1, wherein there are y monomers of formula (1'), such that X is a successive integer from 2 and up to y+1 for each additional monomer (A₂, A₃, A₄, ... A_{y+1});
Ax is selected from molecules comprising at least one functional group G, including from molecules comprising peptides, wherein A₁ and said y Ax are different monomers;
G are free functional groups of peptides A₁ and of A_{X};
q and r(x) represent the total numbers of free functional groups G of A₁ and of said one or more A_{X}, respectively, wherein q and r(x) are independently 1 or more than 1;
L is an organic linker moiety comprising from 1 to 1000 carbons and from 0 to 300 heteroatoms;
D is a free functional group of said type B monomer;
wherein during said step of reacting, said groups D reacts with at least some of said groups G so as to connect said first and second monomers.

2. The method of claim 1, wherein A₁ comprises a bioactive peptide consisting of 2 to 100, preferably 5 to 50 amino acids.

3. The method of claim 1 or 2, wherein A₁ comprises a bioactive peptide comprises an oligo- or polypeptide comprising an immunogenic determinant, preferably a neoepitope.

4. The method of any one of the preceding claims, wherein A₁ and/or A_{X} further comprise a terminal lysine or a peptide tag comprising one or several lysines, wherein at least one of said groups G, respectively, is present on said peptide tag and is an amino side chain group of said lysine or lysines.

5. The method of any one of the preceding claims, wherein said y A_{X} are independently selected from the moieties comprising peptides (as A₁) and further from moieties comprising an adjuvant, wherein said adjuvants may preferably be selected from the group consisting of: Pam₃CSK₄, cGAMP, LPS, flagellin, MPLA, CpG, Poly(I:C), Poly ICLC, Imidazoquinoline, R848 and all kinds of molecular adjuvants.

6. The method of any one of the preceding claims, wherein D is selected from the groups of formula (5), (5') and (6) below: 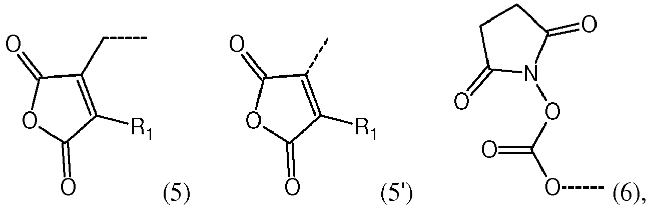 wherein:
R₁ is selected from is selected from H and from aliphatic or aromatic substituents comprising from 1 to 20 carbon atoms and from 0 to 10 heteroatoms, preferably R₁ is C1-C5 alkyl; and,
the dotted line is the single bond by which the group is connected to a carbon or heteroatom of L.

7. The method of any one of the preceding claims, wherein L is a is a moiety of formula (10): 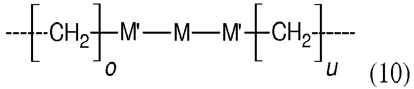 wherein,
o and u are selected, independently, from 0, 1, and 2;
the dotted lines in the structure of formula (10) represent the single bonds by which the carbon of the corresponding CH₂ or M' moiety is connected to said groups D;
o and u are selected, independently, from 0, 1, and 2;
M is a moiety an organic moiety comprising from 1 to 1000 carbons and from 0 to 300 heteroatoms;
M' is selected from the moieties of formula (11), (12), (13), (14) and (15) below: 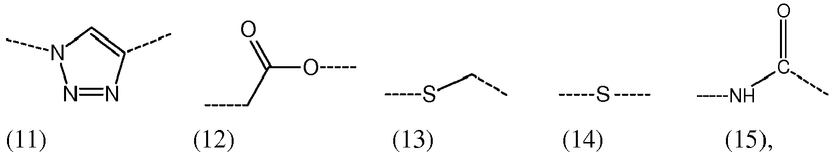 wherein, in formulae (11) to (15), the dotted line on the right represents the bond to M and the dotted line on the left represents the bond to the -CH₂- moiety in formula (10).

8. The method of claim 7, wherein M is a moiety selected from the moieties of formulae (20) - (23) below: 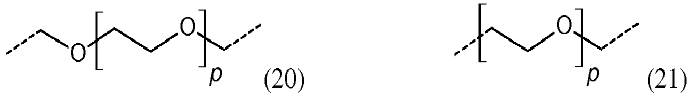 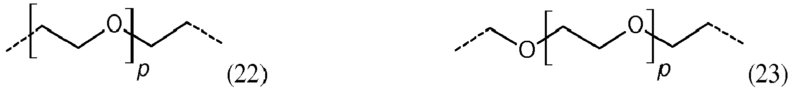 wherein p is 0 or an integer of 1-500, preferably 1-200, more preferably 1- 100, and most preferably 1-20.

9. The method of any one of the preceding claims, wherein L is a moiety of formula (25): 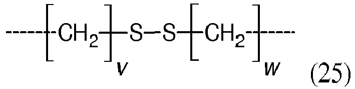 wherein v and w are independently selected from integers of 1-50, preferably 1-10, more preferably 2-5, most preferably v and w are 2.

10. The method of any one of the preceding claims, wherein, if D is a group of formula (5) or (5'), L is a moiety of formula (10), and if D is a moiety of formula (6), L is a moiety of formula (25).

11. The method of any one of the preceding claims, wherein said second monomers (type B or linker monomer) are selected from compounds of formula (28) and (29): 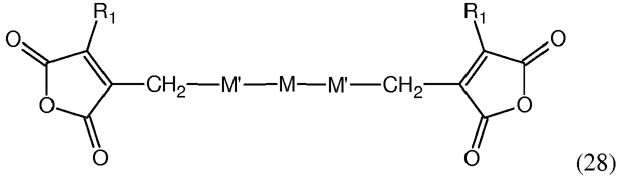 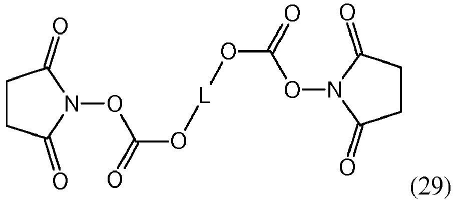 wherein M', M and L are as defined in the preceding claims, preferably in claims 7 and 9, and R₁ is defined as is claim 6.

12. The method of any one of the preceding claims, wherein said second monomers (type B or linker monomer) are selected from compounds of formula (31) and (32): 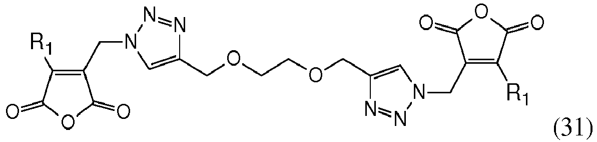 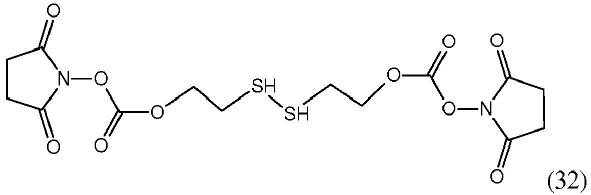 wherein R₁ is as defined in claim 6, preferably C1-C5 alkyl, most preferably methyl.

13. A polymer obtained by the method of any one of the preceding claims.

14. The use of monomers of formulae (2)
D-L-D (2)
and molecules comprising one or more free amino groups for producing polymers that decompose in response to pH, preferably under acidic pH, wherein D is a group of formula (5) as defined in claim 6, and L is a moiety of formula (10) as defined in claim 7.

15. The use of monomers of formulae (2)
D-L-D (2),
wherein D is a group of formula (5) as defined in claim 6, and L is a moiety of formula (10) as defined in claim 7,
for cross-linking bioactive compounds comprising free amino groups.

16. A polymer comprising one or several moieties of formula (40): 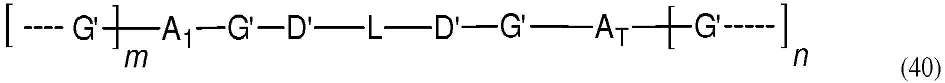 wherein:
A₁, and L are as defined in claim 1 and claims depending on claim 1,
T is 1 or 2, wherein if T is 2, A_{T} is A₂ which is different from A₁;
A₂ is defined as A_{X} in claim 1, with y=1 and X=2;
the moieties -G'-D'- and -D'-G'- are moieties obtained by the reaction of groups D with groups G as defined in claim 1 and further defined in claims depending on claim 1,
m and n independently represent the numbers of groups G of A₁ and A_{T}, respectively, that have reacted with groups D of the second monomer of formula (2), wherein m and n are independently equal or smaller than p and q as defined in claim 1, preferably 100 or less.

17. The polymer of claim 17, wherein -G'-D'- and -D'-G'- are moieties selected from moieties of formula (45) and (46): 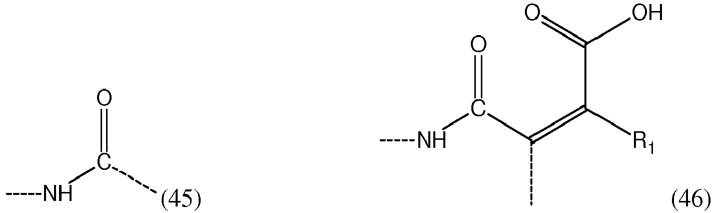 wherein
G' is a derivatized amino group -NH- as appearing in formulae (45) and (46),
wherein the dotted line on the left of formula (45) and (46) represent the bond by which the moiety -G'-D'- and -D'-G'- is connected to A₁ or A_{T}, respectively, and,
wherein the dotted line on the right or on the bottom of formula (45) or (46), respectively, represents the bond by which -G'-D'- and -D'-G'- is connected to L,
and wherein is as defined in claim 6.

18. A compound of formula (2)
D-L-D (2),
wherein D is as defined in claim 6 and L is as defined in claim 7-10.

19. The compound of claim 18, which is a compound of formula (28) or (29) as defined in claim 11 or of formula (31) or (32) as defined in claim 12.
